(19) Europäisches Patentamt
European Patent Office
Office européen des brevets

(11) **EP 2 986 589 B1**

(12) **EUROPÄISCHE PATENTSCHRIFT**

(45) Veröffentlichungstag und Bekanntmachung des Hinweises auf die Patenterteilung:
**14.03.2018 Patentblatt 2018/11**

(21) Anmeldenummer: **14720514.0**

(22) Anmeldetag: **11.04.2014**

(51) Int Cl.:
**C07C 45/75** *(2006.01)*     **C07C 67/39** *(2006.01)*

(86) Internationale Anmeldenummer:
**PCT/EP2014/057380**

(87) Internationale Veröffentlichungsnummer:
**WO 2014/170223 (23.10.2014 Gazette 2014/43)**

(54) **VERFAHREN ZUR HERSTELLUNG VON METHYLMETHACRYLAT**

METHOD FOR PRODUCING METHYL METHACRYLATE

PROCÉDÉ DESTINÉ À LA FABRICATION DE MÉTHYLMÉTHACRYLATE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.04.2013 EP 13002076**

(43) Veröffentlichungstag der Anmeldung:
**24.02.2016 Patentblatt 2016/08**

(73) Patentinhaber: **Evonik Röhm GmbH 64293 Darmstadt (DE)**

(72) Erfinder:
• **KRILL, Steffen 64367 Mühltal (DE)**
• **BALDUF, Torsten 64319 Pfungstadt (DE)**
• **KÖSTNER, Martin 64295 Darmstadt (DE)**
• **GRÖMPING, Matthias 64295 Darmstadt (DE)**
• **LYGIN, Alexander 64347 Griesheim (DE)**
• **BURGHARDT, Rudolf 64287 Darmstadt (DE)**

(56) Entgegenhaltungen:
**EP-A1- 0 092 097     EP-A1- 0 890 569
DE-A1- 2 855 504**

**Beschreibung**

Die vorliegende Erfindung betrifft ein Verfahren zur Herstellung von Methylmethacrylat durch direkte oxidative Veresterung von Methacrolein und die Herstellung von Methacrolein.

Methylmethacrylat wird in großen Mengen zur Herstellung von Polymeren und Copolymeren mit anderen polymerisierbaren Verbindungen eingesetzt. Weiterhin ist Methylmethacrylat ein wichtiger Baustein für diverse, auf Methacrylsäure (MAS) basierender Spezialester, die durch Umesterung mit dem entsprechenden Alkohol hergestellt werden.

Hieraus ergibt sich ein großes Interesse an möglichst einfachen, wirtschaftlichen und umweltschonenden Herstellungsverfahren für diesen Ausgangsstoff.

Methylmethacrylat (MMA) wird heute überwiegend ausgehend von Blausäure und Aceton über das entstehende Acetoncyanhydrin (ACH) als Zentralintermediat hergestellt. Dieses Verfahren hat den Nachteil, dass sehr große Mengen an Ammoniumsulfat erhalten werden, deren Aufbereitung mit sehr hohen Kosten verbunden ist. Weitere Verfahren, die eine andere Rohstoffbasis als ACH verwenden, sind in der einschlägigen Patentliteratur beschrieben und mittlerweile im Produktionsmaßstab realisiert worden. In diesem Zusammenhang werden heute auch C-4 basierte Rohstoffe wie Isobutylen oder tert-Butanol als Edukte verwendet, die über mehrere Verfahrensstufen in die gewünschten Methacrylsäurederivate umgewandelt werden.

Hierbei wird im Allgemeinen Isobutylen oder tert-Butanol zu Methacrolein in einer ersten Stufe oxidiert, welches anschließend zu Methacrylsäure mit Sauerstoff umgesetzt wird. Die erhaltene Methacrylsäure wird nachfolgend mit Methanol in MMA überführt. Nähere Einzelheiten zu diesem Verfahren sind unter anderem in Ullmann's Encyclopedia of Industrial Chemistry 2012, Wiley-VCH Verlag GmbH & Co. KGaA, Weinheim, Methacrylic Acid and Derivatives, DOI:

10.1002/14356007.a16_441.pub2 und in Trends and Future of Monomer-MMA Technologies, SUMITOMO KAGAKU 2004- II dargelegt.

Gemäß einer Abwandlung dieser Herstellungsmethode kann anstatt von einem C4-Baustein, wie Isobutylen, auch von Ethylen ausgegangen werden, welches mit Synthesegas zunächst zu Propanal umgesetzt wird und anschließend mit Formaldehyd zu Methacrolein umgesetzt wird. Das erhaltene Methacrolein wird mit Luft in der Gasphase am heterogenen Kontakt zu Methacrylsäure oxidiert, die mit Methanol zu MMA verestert (Ullmann's Encyclopedia of Industrial Chemistry 2012, Methacrylic Acid from Ethylene und Trends and Future of Monomer-MMA Technologies, SUMITOMO KAGAKU 2004- II). Dieses Verfahren wird von BASF seit 1990 in einer Anlage mit einer Kapazität von 40.000 t/a zur Herstellung von Methacrylsäure durchgeführt. Gemäß dem Artikel von SUMITOMO handelt es sich hierbei um ein Verfahren, welches von BASF für spezifische Bedürfnisse entwickelt wurde, so dass es daher schwierig sei, dieses Verfahren allgemein zur Herstellung von größeren Mengen an MMA einzusetzen.

In einem weiteren Verfahren wird MMA durch Oxidation von Isobutylen oder tert-Butanol mit Luftsauerstoff in der Gasphase am heterogenen Kontakt zu Methacrolein und anschließender oxidativer Veresterungsreaktion von Methacrolein unter Verwendung von Methanol erhalten. Dieses von ASAHI entwickelte Verfahren ist unter anderem in den Druckschriften US 5,969,178 und US 7,012,039 beschrieben. Auch dieses Verfahren wird in dem Artikel von SUMITOMO beschrieben, wobei ausführlich auf die Nachteile dieses Verfahrens hingewiesen wird, die insbesondere in einem hohen Energiebedarf bestehen, was u.a. durch die drucklose Fahrweise bedingt ist.

Problematisch an allen oben dargestellten Verfahren ist insbesondere weiterhin auch die relativ unbefriedigende Ausbeute, hohe Verluste bei den Oxidationsschritten und damit einhergehende $CO_2$ Bildung, und allgemein eine damit einhergehende Nebenproduktbildung, die aufwendige Verfahrensschritte zur Isolierung des Produkts bedingt. So erreichen alle Verfahren, die von Isobutylen oder equivalenten C-4 basierten Rohstoffen wie TBA oder MTBE ausgehen, in der Gasphasenoxidation an einem heterogenen Katalysatorsystem Ausbeuten unterhalb 90%, in der einschlägigen Literatur sind Ausbeuten von unterhalb 85% für die Methacroleinerstellung ausgehend von Isobutylen beschrieben (z.B. Tabelle 5 in Ullmann's Encyclopedia/Sumitomo, siehe oben). Naturgemäß verläuft das Gasphasenverfahren bei moderaten Drücken zwischen 1 bis 2 bar absolut und erzeugt ein Prozessgas, in dem die Produktkomponente nur zu etwa 4-6 Vol-% enthalten ist. Die Isolierung des Wertprodukts vom inerten Gasballast ist entsprechend energetisch aufwendig und verbraucht große Mengen an Kühlenergien sowie Dampf für mehrstufige destillative Aufarbeitungsschritte.

Bei der Herstellung von MMA gemäß den bisher beschriebenen Methoden werden relativ große Mengen an Abfällen, insbesondere an Abgasen oder an Abwässern gebildet, die aufwändig beseitigt werden müssen.

Darüber hinaus werden zur Durchführung einiger der zuvor beschriebenen Verfahren sehr komplexe und damit teure Anlagen benötigt, die mit hohen Investitions- und Unterhaltskosten verbunden sind.

In dem zuvor zitierten Übersichtsartikel von SUMITOMO werden die jeweiligen Nachteile ausführlich dargestellt, so dass hierauf Bezug genommen werden kann.

Darüber hinaus wird in der Patentanmeldung CN 101074192 ein Verfahren zur Herstellung von MMA beschrieben, bei dem zunächst aus Propanal und Formaldehyd bei einer Temperatur im Bereich von 40 bis 45 °C und einer Reaktionszeit im Bereich von 30 bis 100 Minuten Methacrolein gebildet wird, welches anschließend mit Methanol zu MMA oxidiert wird. Ein ähnliches Verfahren wird weiterhin von Yuchao Li et al. "Synthesis of methacrolein by condensation of propionaldehyde with formaldehyde", Advance Materials Research Vols. 396-398 (2012) pp 1094 - 1097 vorgeschlagen. Von einem Arbeiten bei erhöhter Temperatur oder einem Überdruck wird in dieser Druckschrift ausdrücklich abgeraten. Nachteilig an diesem Verfahren ist der hohe Bedarf an Säure und Amin, welche zur Katalyse der Reaktion eingesetzt werden. Hierdurch entstehen große Mengen an Abfallprodukten, da das Amin unter den genannten Bedingungen zu einem beachtlichen Anteil zerstört wird. Eine der Nebenreaktionen, die den Katalysator desaktiviert, ist die Eschweiler-Clarke Reaktion, die zur Bildung von methyliertem tertiären Amin führt, das nicht mehr in der Lage ist, die Mannich Reaktion zu katalysieren (US 4,408,079, Spalte 2, Zeilen 15ff). So wird beispielsweise aus Dimethylamin Trimethylamin.

Wird nun wie bei Li beschrieben, bei oder nahe Normaldruck mit hohen stöchiometrischen Mengen an Katalysatorbase gearbeitet, kommt es zur vermehrten Desaktivierung des Katalysators, was letztlich keine wirtschaftliche Arbeitsweise darstellt. Durch diese Problematik entstehen hohe Kosten, die das beschriebene Verfahren relativ unwirtschaftlich machen. Die durch die drucklose Reaktionsführung bedingte lange Reaktionszeit ist ein weiterer gravierender Nachteil dieser beiden Verfahren.

EP 0 890 569 offenbart ein Verfahren zur Herstellung von Methylmethacrylat durch direkte oxidative Veresterung von Methacrolein mit Methanol. Dabei lehrt die EP 0 890 569 explizit, dass ein niedriger Wassergehalt im Methacrolein von unter 2 Gew%, bevorzugt unter 1 Gew% für die oxidative Veresterung essentiell ist. In den Beispielen sind ausschließlich Umsetzungen mit einem Wassergehalt kleiner 0,8 Gew% aufgeführt. Weiterhin ist es gemäß dieser Lehre wichtig, dass der Gehalt an Verunreinigungen insgesamt gering sein muss. Daher lehrt die EP 0 890 069, dass das Methacrolein in der Gasphase aus Isobutylen oxidativ mit Sauerstoff hergestellt und anschließend aufwendig in einer Kolonne entwässert wird.

EP 0 092 097 und DE 28 55 504 lehren zwar ein alternatives Syntheseverfahren für das Methacrolein in flüssiger Phase. Bei diesem Verfahren wird Propanal mit Formaldehyd umgesetzt. Jedoch entsteht bei diesem Verfahren eine große Menge Wasser, die gemäß der Lehre der EP 0 890 569 ein solches Verfahren ohne aufwendige Aufreinigung als Vorstufe für die oxidative Versterung des Methacroleins zu Methylmethacrylat ungeeignet macht. Neben dem hohen Wassergehalt wirken jedoch auch die in diesem Verfahren eingesetzten Edukte und Nebenprodukte, wie beispielsweise das dimere Methacrolein, in einer oxidativen Verseterung potentiell ausbeutesenkend bzw. schädlich. So enthält das Produkt gemäß der DE 28 55 504 über 5 Gew% eines Aldol-Nebenprodukts, dass in der oxidativen Veresterung schädlich wäre und eine aufwendige Aufreinigung des Methacroleins unumgänglich machen würde.

In Anbetracht des Standes der Technik ist es daher Aufgabe der vorliegenden Erfindung, ein technisch verbessertes Verfahren zur Herstellung von MMA zur Verfügung zu stellen, das nicht mit den Nachteilen herkömmlicher Verfahren behaftet ist.

Insbesondere soll es ermöglicht werden, MMA bei einem geringeren Energiebedarf zu erzeugen. Weiterhin soll das Verfahren sehr umweltschonend durchgeführt werden können, so dass sehr geringe Mengen an Abfällen erhalten werden. Insbesondere ist es Aufgabe der vorliegenden Erfindung, die Gesamtausbeute an MMA bezogen auf die eingesetzten Rohstoffe zu verbessern, beispielsweise durch das Auffinden und Kombinieren von einzelnen Reaktionsschritten mit hoher Produktselektivität.

Des Weiteren sollte das Verfahren mit möglichst wenigen Verfahrensschritten durchgeführt werden können, wobei dieselben einfach und reproduzierbar sein sollten.

Ferner sollte das Verfahren mit relativ einfachen und kostengünstigen Anlagen durchgeführt werden können. Die Anlagen sollten demgemäß mit geringen Investitionskosten verbunden sein. Hierbei sollten die Anlagen einfach zu warten sein und geringe Unterhaltskosten verursachen.

Weitere nicht explizit genannte Aufgaben ergeben sich aus dem Gesamtzusammenhang der nachfolgenden Beschreibung und der Ansprüche.

Gelöst werden die oben erwähnten sowie weitere nicht explizit genannten Aufgaben, die jedoch aus den hierin einleitend diskutierten Zusammenhängen ohne weiteres ableitbar oder erschließbar sind, durch ein Verfahren mit allen Merkmalen des Patentanspruchs 1. Zweckmäßige Abwandlungen des erfindungsgemäßen Verfahrens zur Herstellung von MMA werden in den Unteransprüchen 2 bis 18 unter Schutz gestellt.

Gegenstand der vorliegenden Erfindung ist dementsprechend ein Verfahren zur Herstellung von MMA, umfassend die Schritte:

A) Herstellung von Methacrolein aus Propanal und Formaldehyd und

B) Umsetzung des in Schritt A) erhaltenen Methacroleins in einer oxidativen Veresterungsreaktion zu MMA,

welches dadurch gekennzeichnet ist, dass die beiden Schritte A) und B) in flüssiger Phase bei einem Druck von 2 bis 100 bar erfolgen und Schritt B) in Gegenwart eines heterogenen, Metalle und/oder Metalloxide umfassenden, edelmetallhaltigen Katalysators durchgeführt wird.

Durch das erfindungsgemäße Verfahren gelingt es auf nicht vorhersehbare Weise, ein Verfahren zur Herstellung von MMA bereitzustellen, das nicht mit den Nachteilen herkömmlicher Verfahren behaftet ist. Überraschend wurde dabei festgestellt, dass die Verfahrensschritte A) und B) entgegen der allgemeinen Lehre des Standes der Technik auch ohne eine aufwendige zwischengeschaltete Aufreinigung bzw. Entwässerung des Methacroleins kombinierbar sind und zu hohen Methylmethacrylat-Ausbeuten führen.

Insbesondere kann MMA bei einem geringeren Energiebedarf erzeugt werden. Weiterhin kann das Verfahren sehr umweltschonend durchgeführt werden, wobei relativ geringe Mengen an Abfällen erhalten werden und die Atomökonomie wesentlich erhöht wird.

Insbesondere muss bei dem erfindungsgemäßen Verfahren in keinem der Reaktionsschritte A) und B) zusätzliches Wasser zu dem Reaktionsgemisch zugeführt und anschließend wieder abgetrennt werden, wodurch die Reaktionsvolumina und Ströme insgesamt gering gehalten werden können.

Des Weiteren kann das Verfahren mit relativ wenigen Verfahrensschritten durchgeführt werden, wobei dieselben einfach und reproduzierbar sind und mit höheren Raum-Zeit-Ausbeuten verlaufen.

Ferner ist der Bedarf an Katalysator, insbesondere an organischer Base pro Tonne hergestelltem MMA sehr gering.

Ferner kann das Verfahren mit relativ einfachen und kostengünstigen Anlagen durchgeführt werden. Die Anlagen sind mit geringen Investitionskosten verbunden. Hierbei sind die Anlagen einfach zu warten und verursachen geringe Unterhaltskosten.

In diesem Zusammenhang ist festzuhalten, dass die Anzahl und Größe der Rückströme verglichen mit den Verfahren gemäß dem Stand der Technik reduziert sind

Ferner können die entstehenden Nebenprodukte sehr einfach aus den Reaktionsmischungen abgetrennt werden, so dass das Verfahren insgesamt mit einer hohen Ausbeute durchgeführt werden kann, ohne dass aufwendige Reinigungsschritte notwendig sind.

Schritt A)

Das erfindungsgemäße Verfahren umfasst die Herstellung von Methacrolein. Die hierzu geeigneten Verfahren sind dem Fachmann bekannt und Gegenstand entsprechender Übersichtsartikel, zum Beispiel in Ullmann's Encyclopedia of Industrial Chemistry 2012, Wiley-VCH Verlag GmbH & Co. KGaA, Weinheim, Acrolein and Methacrolein, DOI: 10.1002/14356007.a01_149.pub2.

In der ersten Verfahrensstufe umfasst das erfindungsgemäße Verfahren die Umsetzung von Propanal mit Formalin zu Methacrolein.

Insbesondere geeignet sind die Verfahren, bei denen die Gesamtmenge des während der Verfahrensdurchführung separat zugegebenen Wassers beziehungsweise Wasserdampf nicht größer ist als 100 Mol-%, bevorzugt 50 Mol-%, besonders bevorzugt 30 Mol-% und am meisten bevorzugt 10 Mol-%, jeweils bezogen auf Methacrolein. Am besten geeignet sind Verfahren zur Herstellung von Methacrolein, bei denen im in keinem Reaktionsschritt Wasser separat zur Reaktionsmischung gegeben wird. Ausgenommen hiervon ist das mit den Reaktanden und/oder Katalysatoren eingebrachte Wasser sowie das entstehende Reaktionswasser.

Es ist jedoch überraschend, dass der Wassergehalt weniger relevant, bzw. bis zu Konzentrationen von 5 Gew% für die oxidative Versesterung des Verfahrensschritte B) nahezu nicht störend sind. Damit ist es in Hinblick auf den zitierten Stand der Technik insbesondere überraschend, dass die Methacroleinsynthese aus Propanal und Formaldehyd mit der oxidativen Veresterung kombiniert werden kann, und dabei eine auf eine Entwässerung nach der Verfahrensschritt A) sogar optional verzichtet werden kann.

Die Umsetzung, die über eine Aldol- bzw. Mannichkondensation erreicht wird, ist als solche nicht kritisch. Bevorzugt sind jedoch Verfahren, die sich durch eine hohe Ausbeute und geringe Nebenproduktbildung auszeichnen.

Vorzugsweise werden daher Reaktionen eingesetzt, die eine Selektivität von mindestens 80 %, vorzugweise mindestens 90 % und besonders bevorzugt mindestens 92 % aufweisen, bezogen auf die eingesetzte Menge an Propanal.

Ferner sind Reaktionen bevorzugt, die eine hohe Ausbeute und hohe Umsätze beim einmaligen Durchlaufen der Reaktionszone aufweisen. Bevorzugte Reaktionen zeichnen sich durch eine Ausbeute und Umsätze von mindestens 80 %, vorzugweise mindestens 90 % und besonders bevorzugt mindestens 92 % aus, bezogen auf die eingesetzte Menge an Propanal.

Ferner kann vorgesehen sein, dass die Umsetzung gemäß Schritt A) vorzugsweise mit einem molaren Verhältnis von Propanal zu Formaldehyd im Bereich von 2:1 bis 1:2, besonders bevorzugt 1,5:1 bis 1:1,5 und speziell bevorzugt 1,1:1 bis 1:1,1 erfolgt. Ganz besonders bevorzugt wird ein äquimolares Verhältnis von Propanal zu Formaldehyd eingesetzt. Hierdurch kann, insbesondere bei hohen Umsätzen auf eine Abtrennung und Rückführung von Propanal und/oder Formaldehyd aus der nach der Umsetzung gemäß Schritt A) erhaltenen Mischung verzichtet werden.

Im Allgemeinen werden zur Umsetzung von Propanal mit Formaldehyd Katalysatoren eingesetzt, wobei verschiedene Systeme bekannt sind, die bei einer hohen Selektivität zu einer hohen Ausbeute an Methacrolein führen.

Bevorzugte Verfahren zur Herstellung von Methacrolein, ausgehend von Propanal und Formaldehyd sind unter anderem in den Druckschriften US 7,141,702; DE 32 13 681 A1; US 4,408,079; US 2,848,499; JP 4173757A (JP 19900300135); JP 3069420B2 und EP 0 317 909 A2 beschrieben, wobei die Lehren dieser Druckschriften zu Offenbarungszwecken durch Referenz hierauf in die vorliegende Anmeldung eingefügt werden.

Die Umsetzung von Propanal mit Formaldehyd wird in Gegenwart von Säure, in der Regel anorganischer Säure, organischer Mono-, Di- bzw. Polycarbonsäure, bevorzugt Monocarbonsäure, insbesondere aliphatischer Monocarbonsäure, durchgeführt.

Als Carbonsäuren verwendet man zweckmäßig aliphatische Monocarbonsäuren mit 1 bis 10, vorzugsweise 2 bis 4 Kohlenstoffatomen, oder Di- und Polycarbonsäuren mit 2 bis 10, vorzugsweise 2 und 4 bis 6 Kohlenstoffatomen. Die Dicarbonsäuren und Polycarbonsäuren können aromatische, araliphatische und bevorzugt aliphatische Carbonsäuren sein. Geeignet sind z.B. Essigsäure, Propionsäure, Methoxyessigsäure, n-Buttersäure, iso-Buttersäure, Oxalsäure, Bernsteinsäure, Weinsäure, Glutarsäure, Adipinsäure, Maleinsäure, Fumarsäure. Andere organische Säuren sind prinzipiell ebenfalls verwendbar, in der Regel aber aus Preisgründen weniger zweckmäßig. Als anorganische Säuren werden in der Regel Schwefel- und Phosphorsäure eingesetzt. Es können auch Säuregemische verwendet werden.

Besonders bevorzugt wird zur Umsetzung von Propanal und Formaldehyd mindestens eine organische Säure, besonders bevorzugt Essigsäure eingesetzt.

Der Anteil an Säure beträgt zwischen 0,1 und 20, vorteilhaft von 0,5 bis 10, bevorzugt 1 bis 5 Mol-%, bezogen auf Propanal.

Die Umsetzung von Propanal mit Formaldehyd wird in Gegenwart von organischen Basen, vorzugsweise Aminen, besonders bevorzugt sekundären Aminen durchgeführt. Als Amine kommen vorzugsweise solche der Formel $R^1R^2NH$, in Betracht, bei denen $R^1$ und $R^2$ gleich oder verschieden sind und jeweils einen Alkylrest mit 1 bis 10, vorteilhaft 1 bis 8, insbesondere 1 bis 4 Kohlenstoffatomen, die noch durch Ether-, Hydroxy-, sekundäre, tertiäre Aminogruppen, insbesondere durch 1 bis 2 dieser Gruppen, substituiert sein können, einen Aralkylrest mit 7 bis 12 Kohlenstoffatomen, einen Cycloalkylrest mit 5 bis 7 Kohlenstoffatomen bedeuten, $R^1$ und $R^2$ auch mit dem benachbarten Stickstoff Glieder eines heterocyclischen, vorteilhaft 5 bis 7-gliedrigen Ringes, der noch ein weiteres Stickstoffatom und/oder ein Sauerstoffatom enthalten und durch Hydroxyalkyl- oder Alkylgruppen mit 1 bis 4 Kohlenstoffatomen substituiert sein kann, bezeichnen können.

Als Amine kommen beispielsweise in Betracht: Dimethylamin, Diethylamin, Methylethylamin, Methylpropylamin, Dipropylamin, Dibutylamin, Di-iso-propylamin, Di-iso-butylamin, Methyl-iso-propylamin, Methyl-iso-butylamin, Methyl-sek.butylamin, Methyl-(2-methylpentyl)-amin, Methyl-(2-ethylhexyl)-amin, Pyrrolidin, Piperidin, Morpholin, N-Methylpiperazin, N-Hydroxyethyl-piperazin, Piperazin, Hexamethylenimin, Diethanolamin, Methylethanolamin, Methylcyclohexylamin, Methylcyclopentalamin, Dicyclohexylamin oder entsprechende Gemische.

Ferner kann vorgesehen sein, dass mindestens eines der eingesetzten Amine keine Hydroxygruppe aufweist. Besonders bevorzugt beträgt der Anteil an Aminen mit mindestens einer Hydroxygruppe höchstens 50 Gew-%, vorzugsweise höchstens 30 Gew-%, und besonders bevorzugt höchstens 10 Gew-%, bezogen auf das Gewicht der eingesetzten Amine

Der Anteil an organischer Base, vorzugsweise sekundären Aminen beträgt zwischen 0,1 und 20, vorteilhaft von 0,5 bis 10, bevorzugt 1 bis 5 Mol-%, bezogen auf Propanal.

Das Verhältnis der Äquivalente Amin zu Säure wird vorzugsweise so gewählt, dass im Reaktionsgemisch vor der Reaktion ein pH-Wert von 2,5 bis 9 resultiert.

Ferner kann vorgesehen sein, dass das molare Verhältnis von Säure zu organischer Base, vorzugsweise Amin im Bereich von 20:1 bis 1:20, bevorzugt im Bereich von 10:1 bis 1:10, besonders bevorzugt im Bereich von 5:1 bis 1:5 und speziell bevorzugt im Bereich von 2:1 bis 1:2 liegt.

Die Reaktionstemperatur der Umsetzung von Propanal mit Formaldehyd am Austritt der Reaktionszone liegt zwischen 100 und 300 °C, vorzugsweise 130 und 250 °C, bevorzugt von 140 bis 220 °C, insbesondere 150 bis 210 °C.

Der Reaktionsdruck liegt im Bereich von 2 bis 300, bevorzugt bei 5 bis 250, besonders bevorzugt von 10 bis 200 bar, vorteilhaft von 15 bis 150 bar, bevorzugt 20 bis 100 bar und insbesondere 40 bis 80 bar. Druck und Temperatur werden so eingestellt, dass die Umsetzung stets unterhalb des Siedepunktes des Reaktionsgemisches erfolgt, die Reaktion also in flüssiger Phase verläuft.

Alle Druckangaben im Rahmen der vorliegenden Anmeldung erfolgen als absoluter Druck in der Maßeinheit bar.

Die Verweilzeit bzw. Reaktionszeit beträgt vorzugsweise höchstens 25, zweckmäßig 0,01 bis 25, vorteilhaft 0,015 bis 10, bevorzugt 0,03 bis 2 Minuten. Besonders bevorzugt liegt die Verweilzeit bzw. Reaktionszeit im Bereich von 0,1 bis 300 Sekunden, speziell bevorzugt im Bereich von 1 bis 30 Sekunden. Als Reaktor wird bei Verweilzeiten unter 10 Minuten vorteilhaft ein Rohrreaktor eingesetzt. Die Verweilzeit bezieht sich hierbei auf die Zeit, über die die Reaktionsmischung umgesetzt wird. Hierbei liegen sämtliche Komponenten bei Reaktionsdruck und Temperatur vor, so dass diese Zeit aus der Strecke zwischen Mischpunkt und dem Entspannungspunkt berechnet werden kann. Der Entspannungspunkt ist der Punkt, bei dem die Mischung vom Reaktionsdruck auf einen Druck unterhalb von 5 bar gebracht wird.

In den Reaktionsgemischen können neben Wasser zusätzlich organische Lösungsmittel wie z.B. Propanol, Dioxan, Tetrahydrofuran, Methoxyethanol enthalten sein.

Ferner kann vorgesehen sein, dass die Umsetzung von Propanal mit Formaldehyd zu Methacrolein gemäß Schritt A) in Gegenwart von bevorzugt mindestens 0,1 Gew-%, vorzugsweise mindestens 0,2 Gew-% und besonders bevorzugt mindestens 0,5 Gew-% Methanol bezogen auf Formalin erfolgt. Trotz dieser höheren Methanol-Konzentrationen kann aufgrund der erfindungsgemäßen Reaktionsführung für den Folgeschritt B) auf eine aufwendige Abtrennung von Methanol auf der Stufe der Formalinherstellung und/oder der Methacroleinreinigung verzichtet werden.

Gemäß einer besonderen Ausgestaltung kann Formaldehyd und Propanal gemischt werden, bevor diese Edukte auf Reaktionsdruck und/oder Temperatur gebracht werden.

Die Reaktion kann wie folgt durchgeführt werden: Ein Gemisch von Propanal, Amin, Formaldehyd und zweckmäßig Wasser und/oder Säure und/oder Base wird während der Reaktionszeit bei der Reaktionstemperatur und dem Reaktionsdruck gehalten.

In einer bevorzugten Ausführungsform kann ein Gemisch (zweckmäßig äquimolares Gemisch) aus Formaldehyd und Propanal über einen Wärmetauscher auf die gewünschte Reaktionstemperatur erhitzt und einem Rohrreaktor zugeleitet werden. In dieses Gemisch kann eine Katalysatorlösung (Lösung des sekundären Amins und einer Säure, zweckmäßig in $H_2O$) am Reaktoreingang eingedüst werden, die gegebenenfalls über einen Wärmetauscher ebenfalls auf die Reaktionstemperatur erwärmt wird. Die stark exotherme Reaktion setzt ein und das Reaktionsgemisch erwärmt sich weiter. Der Druck, unter dem die Reaktion abläuft, wird vorzugsweise durch ein Druckhalteventil am Reaktorausgang auf solchen Werten gehalten, dass das Reaktionsgemisch auch bei hohen Temperaturen im Reaktor während der Reaktionszeit noch flüssig bleibt. Nach der Umsetzung kann das Reaktionsgemisch auf Normaldruck entspannt und aufgearbeitet werden. Bei der Herstellung von Methacrolein aus Propanal und Formaldehyd wird bevorzugt das Reaktionsgemisch einer Kolonne zugeleitet und dort mit Wasserdampf gestrippt. Das Methacrolein verlässt zusammen mit Wasser die Kolonne am Kopf. Das Gemisch wird kondensiert und über ein Phasentrenngefäß in eine obere und eine untere Phase getrennt. Die obere Phase enthält das Methacrolein und wird in einer oxidativen Veresterung gemäß Schritt B) zu MMA umgesetzt. Die untere Phase besteht hauptsächlich aus Wasser. Vorzugsweise kann sie zur Entfernung des noch darin gelösten Methacroleins zumindest teilweise wieder in die Kolonne zurückgeführt werden.

Die wässrige Katalysatorlösung kann am Sumpf der Kolonne zusammen mit dem bei der Reaktion gebildeten Wasser und dem Wasser der Formaldehydlösung abgezogen werden. Für die Weiterverarbeitung kann, wenn sehr wenig Amin und/oder Säure eingesetzt wird und deshalb die Rückführung des Katalysators nicht mehr lohnt, die Sumpfflüssigkeit verworfen werden.

Bei größeren Amin- und/oder Säurekonzentrationen im Sumpfablauf kann aber auch Wasser teilweise destillativ abgetrennt und die Katalysatorlösung wieder in den Reaktor zurückgeführt werden. Außerdem ist es möglich, den Sumpfablauf so in zwei Teilströme aufzuteilen, dass ein Teilstrom gerade die Menge an Wasser mit sich führt, die bei der Reaktion gebildet und mit den Ausgangsstoffen eingegeben wurde. Dieser Teilstrom wird dann ausgeschleust und der restliche Anteil in den Reaktor zurückgeführt. Wässriger Formaldehyd und Propanal können auch getrennt vorgeheizt und dem Reaktor zugefahren werden.

Das zur Herstellung von Methacrolein eingesetzte Propanal kann kommerziell in großem Maßstab erhalten werden. Vorzugsweise kann diese Verbindung durch Umsetzung von Ethylen mit Kohlenmonoxid (CO) und Wasserstoff ($H_2$) erhalten werden. Die hierfür im Allgemeinen durchgeführte Hydroformulierungsreaktion ist allgemein bekannt, wobei hierfür auf Standardliteratur, beispielsweise Kirk-Othmer Encyclopedia of Chemical Technology, John Wiley & Sons, Inc., OXO Process und Franke et al., Applied Hydroformylation, dx.doi.org/10.1021/cr3001803, Chem. Rev. 2012, 112, 5675-5732 verwiesen wird, wobei diese Druckschriften zu Offenbarungszwecken durch Referenz hierauf in die vorliegende Anmeldung eingefügt wird.

Im Allgemeinen werden für diese Reaktion Katalysatoren eingesetzt. Zu den bevorzugten Katalysatoren zählen insbesondere Verbindungen, die Rhodium, Iridium, Palladium und/oder Cobalt umfassen, wobei Rhodium besonders bevorzugt ist.

Gemäß einer besonderen Ausführungsform können insbesondere Komplexe, die mindestens eine phosphorhaltige Verbindung als Ligand umfassen, zur Katalyse eingesetzt werden. Bevorzugte phosphorhaltige Verbindungen umfassen aromatische Gruppen und mindestens ein, besonders bevorzugt zwei Phosphoratome. Zu den phosphorhaltigen Verbindungen zählen insbesondere Phosphine, Phosphite, Phosphinite, Phosphonite. Beispiele für Phosphine sind Triphenylphosphin, Tris(p-tolyl)phosphin, Tris(m-tolyl)phosphin, Tris(o-tolyl)phosphin, Tris(p-methoxyphenyl)phosphin, Tris(p-dimethylaminophenyl)phosphin, Tricyclohexylphosphin, Tricyclopentylphosphin, Triethylphosphin, Tri-(1-naphthyl)phosphin, Tribenzylphosphin, Tri-n-butylphosphin, Tri-t-butylphosphin. Beispiele für Phosphite sind Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2,4-di-t-butylphenyl)phosphit, Tris(2-t-butyl-4- methoxyphenyl)phosphit, Tris(2-t-butyl-4-methylphenyl)phosphit, Tris(p-kresyl)phosphit. Beispiele für Phosphonite sind Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl- und/oder Arylreste oder Halogenatome ersetzt sind. Gängige Phosphinitliganden sind Diphenyl(phenoxy)phosphin und dessen Derivate Diphenyl(methoxy)phosphin und Diphenyl(ethoxy)phosph in.

Katalysatoren und Liganden zur Hydroformylierung werden beispielsweise in WO 2010/030339 A1, WO 2008/071508 A1, EP 982 314 B1, WO 2008/012128 A1, WO 2008/006633 A1, WO 2007/036424 A1, WO 2007/028660 A1, WO 2005/090276 A1 dargelegt, wobei auf diese Druckschriften zu Offenbarungszwecken verwiesen wird und die darin offenbarten Katalysatoren und Liganden in diese Anmeldung eingefügt werden. In diesen Druckschriften werden auch Reaktionsbedingungen dargelegt, die ebenfalls in die vorliegende Anmeldung aufgenommen werden.

Zur Hydroformylierung von Ethen werden Kohlenmonoxid und Wasserstoff üblicherweise in Form eines Gemisches, dem sogenannten Synthesegas, eingesetzt. Die Zusammensetzung des zur Hydroformylierung eingesetzten Synthesegases kann in weiten Bereichen variieren. Das molare Verhältnis von Kohlenmonoxid und Wasserstoff beträgt in der Regel 2:1 bis 1:2, insbesondere etwa 45:55 bis 50:50.

Die Temperatur bei der Hydroformylierungsreaktion liegt im Allgemeinen in einem Bereich von etwa 50 bis 200 °C, vorzugsweise etwa 60 bis 190 °C, insbesondere etwa 90 bis 190 °C. Die Umsetzung wird vorzugsweise bei einem Druck im Bereich von etwa 5 bis 700 bar, vorzugsweise 10 bis 200 bar, insbesondere 15 bis 60 bar durchgeführt. Der Reaktionsdruck kann in Abhängigkeit von der Aktivität des eingesetzten Hydroformylierungskatalysators variiert werden.

Geeignete druckfeste Reaktionsapparaturen für die Hydroformylierung sind dem Fachmann bekannt. Dazu zählen die allgemein üblichen Reaktoren für Gas-flüssig-Reaktionen, wie z. B. Gasumlaufreaktoren, Blasensäulen etc., die gegebenenfalls durch Einbauten unterteilt sein können.

Weitere bevorzugte Ausgestaltungen einer Hydroformylierungsreaktion sind unter anderem in EP 1 294 668 B1 dargelegt, wobei der Inhalt dieser Druckschrift durch Referenz hierauf in die vorliegende Anmeldung eingefügt wird.

Gemäß einer besonders bevorzugten Ausführungsform kann die Herstellung von Methacrolein aus Propanal und Formaldehyd in einer Tandemreaktion erfolgen wobei Propanal durch die Umsetzung von Ethylen, Kohlenmonoxid und Wasserstoff erhalten und unmittelbar mit Formaldehyd umgesetzt wird. Dieses Verfahren wird ausführlich von Deshpande et al., Biphasic catalysis for a selective oxo-Mannich tandem synthesis of methacrolein, Journal of Molecular Catalysis A: Chemical 211 (2004) 49-53, doi:10.1016/j.molcata.2003.10.010 und in US 7,141,702 B2 beschrieben, wobei diese Druckschriften zu Offenbarungszwecken in die vorliegende Anmeldung durch Referenz hierauf eingefügt werden.

Schritt B)

Erfindungsgemäß wird das in Schritt A) erhaltene Methacrolein in einer direkten oxidativen Veresterungsreaktion zu MMA umgesetzt.

Unter einer direkten oxidativen Veresterungsreaktion wird im Rahmen der vorliegenden Erfindung ein Verfahren verstanden, bei dem Methacrolein in Gegenwart von Methanol und einem Oxidationsmittel, vorzugsweise Sauerstoff, direkt, das heißt ohne die Bildung von großen Mengen an Methacrylsäure, zu MMA umgesetzt wird.

In Verfahren die beispielsweise von BASF durchgeführt werden, wird im Gegensatz hierzu Methacrolein zunächst zu Methacrylsäure oxidiert, welches anschließend in einem weiteren Reaktionsschritt mit Methanol zu MMA verestert wird.

Bei dem Verfahren gemäß BASF können aufgrund von Methanol, welches zur Stabilisierung oder durch das Herstellungsverfahren in dem eingesetzten Formaldehyd enthalten ist, ebenfalls geringe Mengen an MMA während der Oxidation von Methacrolein gebildet werden. Dieses MMA kann jedoch mit kostengünstigen Aufreinigungsverfahren nicht von rückgeführtem Methacrolein getrennt werden und zersetzt sich bei den gewählten Bedingungen im Allgemeinen, so dass die Endausbeute an MMA durch Methanol, welches im Formaldehyd enthalten ist, verringert wird. Als Lösung dieses Problems muss Formaldehyd aufwendig von Methanol befreit werden. Alternativ kann das in der zur Rückführung vorgesehenen Zusammensetzung enthaltene MMA zwar durch einen Reinigungsschritt abgetrennt werden, beide Lösungen sind jedoch mit einem großen Aufwand verbunden, der sich aufgrund der relativ geringen Mengen an gewonnenem MMA nicht lohnt.

Im Allgemeinen entstehen bei der Oxidation von Methacrolein bei einer oxidativen Veresterungsreaktion gemäß Schritt B) des Verfahrens der vorliegenden Erfindung höchstens 30 Gew-%, bevorzugt höchstens 15 Gew-%, besonders bevorzugt höchstens 5 Gew-% Methacrylsäure.

Eine oxidative Veresterungsreaktion wird mit einem Oxidationsmittel durchgeführt, wobei hierzu bevorzugt Sauerstoff ($O_2$) eingesetzt wird. Aus Kostengründen kann vorzugsweise Luft eingesetzt werden, die unterschiedliche Anteile an Sauerstoff umfassen kann, ohne dass dies für die vorliegende Erfindung kritisch wäre.

Weiterhin wird zur Durchführung einer Reaktion gemäß Schritt B) mindestens ein heterogener Oxidationskatalysator eingesetzt, die die vorstehend näher definierte Oxidationsreaktion selektiv beschleunigen. Geeignete Katalysatoren sind in der Fachwelt weithin bekannt und beispielsweise in den Druckschriften EP 0 857 512 A1, EP 1 393 800 A1, EP 2 177 267 A1, und EP 2 210 664 A1, dargelegt, wobei auf diese Druckschriften zu Offenbarungszwecken verwiesen wird und die darin offenbarten Katalysatoren in diese Anmeldung eingefügt werden. In diesen Druckschriften werden auch Reaktionsbedingungen dargelegt, die ebenfalls in die vorliegende Anmeldung aufgenommen werden.

Vorzugsweise umfassen heterogene Oxidationskatalysatoren mindestens ein Edelmetall und/oder mindestens ein Metalloxid. Hierbei sind Oxidationskatalysatoren bevorzugt, die Gold und/oder Palladium und/oder Ruthenium und/oder Rhodium und/oder Silber umfassen. Besonders bevorzugt sind Gold- und/oder Palladiumhaltige Katalysatoren.

Zu den geeigneten Katalysatoren zur Durchführung des vorliegenden Verfahrens zählen unter anderem Palladiumkatalysatoren, die vorzugsweise Palladium und Blei enthalten und im Allgemeinen auf einem Träger eingesetzt werden.

Außerdem kann ein Palladiumkatalysator auch mindestens eine Verbindung enthalten, die aus der Gruppe ausgewählt ist, die aus einer Alkalimetallverbindung und einer Erdalkalimetallverbindung besteht. Es ist bevorzugt, dass ein Palladiumkatalysator 0,01 bis 30 Gew-%, vorteilhafter 0,01 bis 5 Gew-% mindestens einer Verbindung enthält, die aus der Gruppe ausgewählt ist, die aus einer Alkalimetallverbindung und einer Erdalkalimetallverbindung besteht.

Das Einbringen der Alkalimetallverbindung und/oder der Erdalkalimetallverbindung in den Katalysator kann durch ein Verfahren durchgeführt werden, bei dem eine solche Verbindung zu einer Lösung gegeben wird, die eine Palladiumverbindung und/oder eine Bleiverbindung enthält, und ein Träger mit der Lösung behandelt wird, wodurch die Alkalimetallverbindung und/oder die Erdalkalimetallverbindung zusammen mit der Palladiumverbindung und/oder der Bleiverbindung auf den Träger adsorbiert wird oder anhaftet. Alternativ dazu kann ein Träger, der eine darauf aufgetragene Alkalimetallverbindung und/oder eine Erdalkalimetallverbindung enthält, zur Herstellung eines Katalysators eingesetzt werden. Statt des Einsatzes eines Trägers kann eine Lösung, die eine Alkalimetallverbindung und/oder eine Erdalkalimetallverbindung enthält, zur Reaktionsmischung bei der Umsetzung gemäß Schritt B) gegeben werden.

Hinsichtlich der Menge des von dem Träger getragenen Palladiums gibt es keine besondere Einschränkung, die Menge ist jedoch vorzugsweise 0,1 bis 20 Gew-%, stärker bevorzugt 1 bis 10 Gew-%, bezogen auf das Gewicht des Trägers. Hinsichtlich der Menge des von dem Träger getragenen Bleis gibt es keine Einschränkung, die Menge ist jedoch vorzugsweise 0,05 bis 17 Gew-%, stärker bevorzugt 0,45 bis 8,5 Gew-%, bezogen auf das Gewicht des Trägers. Das Atomverhältnis von Palladium zu Blei ist vorzugsweise im Bereich von 3:0,7 bis 3:1,3, stärker bevorzugt im Bereich von 3:0,9 bis 3:1,1.

Nachteilig an den zuvor beschriebenen Palladiumkatalysatoren ist, dass das üblich enthaltene Blei Umweltschäden verursachen kann, so dass Abwässer aufwendig aufbereitet werden müssen, um Bleireste abzutrennen.

In einer alternativen Ausführungsform wird ein Katalysator zur oxidativen Veresterung eingesetzt, der möglichst geringe Mengen an Blei aufweist. Danach kann die oxidative Veresterungsreaktion gemäß Schritt B) unter Verwendung eines Katalysators durchgeführt werden, dessen Bleigehalt bevorzugt höchstens 20 Gew-%, vorzugsweise höchstens 10 Gew-%, speziell bevorzugt höchstens 5 Gew-%, besonders bevorzugt höchstens 2 Gew-% und ganz besonders bevorzugt höchstens 1 Gew-% beträgt. Gemäß einer speziell bevorzugten Ausführungsform wird in Schritt B) ein Katalysator eingesetzt, der bevorzugt höchstens 1,5 Gew-%, vorzugsweise höchstens 1 Gew-%, speziell bevorzugt höchstens 0,5 Gew-%, besonders bevorzugt höchstens 0,2 Gew-% und ganz besonders bevorzugt höchstens 0,1 Gew-% Blei umfasst. Weiterhin kann in Schritt B) ein Katalysator eingesetzt werden, der keine messbaren Anteile an Blei umfasst.

Ferner kann vorgesehen sein, dass die oxidative Veresterungsreaktion gemäß Schritt B) unter Verwendung eines Katalysators durchgeführt wird, der eines oder mehrere Metalle enthält, ausgewählt aus der Gruppe bestehend aus Gold und/oder Palladium und/oder Ruthenium und/oder Rhodium und/oder Silber. Vorzugsweise liegen diese Metalle als ultrafein verteilte Metalle vor, also in Form von Nanopartikeln, die auf einen Träger aufgebracht sind. Bevorzugt weisen die Metallpartikel einen durchschnittlichen Teilchendurchmesser von höchstens 20 nm, bevorzugt höchstens 10 nm besonders bevorzugt 5 nm auf, wobei sich dieser Wert auf ein durch TEM (Transmission Electron Microscope) bestimmtes Zahlenmittel bezieht. Der "durchschnittliche Teilchendurchmesser" der Partikel berechnet sich aus dem Durchmesser von 100 Teilchen, die aus 120 Teilchen ausgewählt sind, wobei die zehn größten Teilchen und die zehn kleinsten Teilchen der 120 Teilchen nicht berücksichtigt werden.

Neben Gold- und/oder Palladium- und/oder Ruthenium- und/oder Rhodium- und/oder Silberpartikeln kann ein bevorzugter Katalysator weitere katalytisch aktive Bestandteile umfassen. Zu den weiteren katalytisch aktiven Bestandteilen gehören unter anderem Magnesium, Scandium, Yttrium, Lanthan und andere Lanthanoide mit den Ordnungszahlen 58 bis 71, Titan, Zirkonium, Hafnium, Vanadium, Niobium, Tantal, Chrom, Molybdän, Wolfram, Mangan, Rhenium, Eisen, Osmium, Kobalt, Iridium, Nickel, Platin, Kupfer, Zink, Cadmium, Aluminium, Gallium, Indium, Thallium, Germanium, Zinn, Blei, Antimon, Bismut, welche jeweils in metallischer und/oder oxidierter Form (z.B. als Oxide, Hydroxide, Salze) vorliegen können. Diese weiteren katalytisch aktiven Bestandteile liegen in Form von Partikeln vor, die vorzugsweise einen durchschnittlichen Teilchendurchmesser von höchstens 20 nm, bevorzugt höchstens 10 nm, besonders bevorzugt höchstens 5 nm aufweisen. Hierbei können die Gold- und/oder Palladium- und/oder Ruthenium- und/oder Rhodium- und/oder Silberpartikel und die Partikel mit weiteren katalytisch aktiven Bestandteilen gemeinsam oder getrennt, insbesondere in legierter oder nicht legierter Form, auf dem Träger vorliegen. Vorzugsweise umfassen die Gold- und/oder Palladium- und/oder Ruthenium- und/oder Rhodium- und/oder Silberpartikel die weiteren katalytisch aktiven Bestandteile.

Der Anteil der katalytisch wirksamen Partikeln des Katalysators kann in weiten Bereichen variieren. Vorzugsweise liegen die Anteile der katalytisch wirksamen Partikel im Bereich von etwa 0,01 bis 20 Gewichtsteile, besonders bevorzugt im Bereich von 0,1 bis 10 Gewichtsteile, pro 100 Gewichtsteile des Katalysators.

Falls der Katalysator neben Goldpartikeln weitere Elemente als katalytisch aktive Komponenten umfasst, kann das Atomverhältnis von Gold zur Summe der weiteren Elemente im Bereich von 1:0,001-1:1000, vorzugsweise von 1:0,01-1:100, besonders bevorzugt 1:0.1-1:10 und speziell bevorzugt 1:0.2-1:5 liegen.

Ferner kann vorgesehen sein, dass die oxidative Veresterungsreaktion gemäß Schritt B) unter Verwendung eines nickelhaltigen Katalysators durchgeführt wird. Vorzugsweise umfassen nickelhaltige Katalysatoren Anteile an einem Edelmetall, vorzugsweise Gold. Vorzugsweise umfassen nickelhaltige Katalysatoren Nickeloxid, welches in Kombination mit Nickel, Palladium, Platin, Ruthenium, Gold, Silber und/oder Kupfer eingesetzt wird. Vorzugsweise liegt das Atomverhältnis von $NiO_x$ zu $(NiO_x + X)$ im Bereich von 0,20 bis 0,99, bevorzugt 0,30 bis 0,90 und besonders bevorzugt 0,50 bis 0.90 wobei X ausgewählt ist aus Nickel, Palladium, Platin, Ruthenium, Gold, Silber und/oder Kupfer, wobei Gold besonders bevorzugt ist. Hierbei liegt in dieser Formel $NiO_x$ als Oxid vor, während X in metallischer Form vorhanden ist.

Nickeloxid ($NiO_x$) kann beispielsweise als $Ni_2O$, $NiO$, $NiO_2$, $Ni_3O_4$ oder $Ni_2O_3$ vorliegen.

Das Nickeloxid und weitere Komponenten, insbesondere metallische Komponenten, wie Nickel, Palladium, Platin, Ruthenium, Gold, Silber und/oder Kupfer können hierbei bevorzugt als Nanopartikel mit einer Größe im Bereich von 2 bis 15 nm, vorzugsweise 2 bis 10 nm und besonders bevorzugt 2 bis 6 nm eingesetzt werden, wobei sich dieser Wert auf ein durch TEM (Transmission Electron Microscope) bestimmtes Zahlenmittel bezieht, wie dies zuvor näher definiert wurde. Die Nanopartikel werden bevorzugt auf einem Träger fixiert.

Die zuvor beschriebenen Katalysatoren werden im Allgemeinen auf einem Träger aufgebracht, wobei diese Träger Metalloxide (wie z. B. Siliciumdioxid, Aluminiumoxid, Titanoxid, Zirkonoxid, oder Magnesiumoxid), gemischte Oxide (wie Siliciumdioxid-Aluminiumoxid, Titandioxid-Siliciumdioxid oder Siliciumdioxid-Magnesiumoxid), Zeolithe (wie ZSM-5), mesoporösen Silikate (wie MCM-41), natürliche Mineralien (wie Ton, Diatomeenerde oder Bims), Kohlenstoff-Materialien (wie z. B. Aktivkohle oder Graphit) umfassen können. Vorzugsweise werden anorganische Träger auf Basis von Oxiden eingesetzt.

Besonders günstig ist die Verwendung eines anorganischen Trägers auf Basis von Oxiden, welcher Silizium, Lithium, Natrium, Kalium, Magnesium, Calcium, Scandium, Yttrium, Lanthan und andere Lanthanoide mit den Ordnungszahlen 58 bis 71, Titan, Zirkonium, Hafnium, Vanadium, Niobium, Tantal, Chrom, Molybdän, Wolfram, Mangan, Rhenium, Eisen, Ruthenium, Osmium, Kobalt, Rhodium, Iridium, Nickel, Platin, Palladium, Kupfer, Silber, Zink, Cadmium, Bor, Aluminium, Gallium, Indium, Thallium, Germanium, Zinn, Blei, Antimon, Bismut und/oder Tellur umfasst.

Ein bevorzugter Träger auf Basis von Oxiden umfasst Siliziumoxid als Hauptkomponente und ein oder mehrere Mitglieder der Gruppe bestehend aus Lithium, Natrium, Kalium, Magnesium, Calcium, Scandium, Yttrium, Lanthan und andere Lanthanoide mit den Ordnungszahlen 58 bis 71, Titan, Zirkonium, Hafnium, Vanadium, Niobium, Tantal, Chrom, Molybdän, Wolfram, Mangan, Eisen, Kobalt, Nickel, Kupfer, Silber, Zink, Cadmium, Aluminium, Gallium, Indium, Thallium, Germanium, Zinn, Blei, Antimon und Bismut.

Es bestehen keine besonderen Beschränkungen bezüglich des Verfahrens zur Herstellung der oben erwähnten anorganischen Träger auf Basis von Oxiden, und jedes bekannte Herstellungsverfahren kann eingesetzt werden. Beispiele umfassen Imprägnieren, Copräzipitation, Ionenaustausch, Gasphasenabscheidung, Kneten oder Hydrothermalsynthese.

Vorzugsweise wird ein poröser Träger eingesetzt. Besonders bevorzugt beträgt die spezifische Oberfläche (BET-Verfahren) in der Regel mindestens 50 $m^2$/g, vorzugsweise mindestens 100 $m^2$/g.

Das Verfahren zur Beladung des Trägers mit den katalytisch aktiven Bestandteilen unterliegt keinen besonderen Restriktionen. Geeignet sind unter anderem Copräzipitation, Abscheidungsfällung, Imprägnierung oder Dampfphasenabscheidung.

Die zuvor dargelegten Katalysatoren auf Basis von Gold und/oder Nickeloxid sind gegenüber den Palladiumkatalysatoren bevorzugt. Vorzugsweise können Nickelhaltige und goldhaltige Katalysatoren bleifrei ausgestaltet werden.

Vorzugsweise kann vorgesehen sein, dass der Wassergehalt der in Schritt B) zur oxidativen Veresterung eingesetzten Reaktionsmischung vorzugsweise höchstens 10 Gew% und bevorzugt höchstens 5 Gew% beträgt.

Diese niedrigen Anteile an Wasser können optional durch den Einsatz eines Phasentrenners erzielt werden, wobei der Wassergehalt der Methacroleinphase temperaturbedingt variieren kann. Vorzugsweise wird die nach der Umsetzung von Formaldehyd mit Propanal erhaltene Reaktionsmischung demgemäß auf eine Temperatur abgekühlt, bei der sich der Wassergehalt in der Methacroleinphase auf die genannten Werte einstellt. Vorzugsweise kann die Temperatur im Phasentrenner zwischen 0 und 50 °C, vorzugsweise 5 bis 30 °C und besonders bevorzugt 10 bis 25 °C eingestellt werden. Eine Abtrennung des Wassers ist jedoch nur bei besonders hohen Wassergehalten von über 10 Gew% insoweit nötig, dass eine deutliche Steigerung der Raum-Zeit-Ausbeute realisiert werden kann. Bei Wassergehalten von über 5 Gew% kann mittels der Wasserabtrennung auf einen Gehalt unter 5 Gew% eine leichte Ausbeutensteigerung des Methylmethacrylats realisiert werden.

Es wurde genauso überraschend festgestellt, dass auch mit einem Restgehalt an Edukten oder Nebenprodukten aus Verfahrensschritt A) entgegen im Stand der Technik vorliegenden Vorurteilen hohe Methylmethacrylat-Raum-Zeit-Ausbeuten realisiert werden können. So werden zwar Propanal, Formaldehyd und dimeres Methacrolein zu Methylpropionat, Methylformiat und zum Methylester des oxidierten dimeren Methacroleins umgesetzt. Dabei stören diese Komponenten den Gesamtprozess jedoch nur in Bezug auf diese Nebenproduktbildung. Somit ist die Gesamtausbeute an MMA überraschend sehr hoch und die genannten Nebenprodukte lassen sich in der Aufarbeitung des MMA leicht abtrennen.

Es ist auch bevorzugt, dass der Gehalt an Methacrolein in der in Schritt B) zur oxidativen Veresterung eingesetzten Reaktionsmischung mindestens 5, bevorzugt mindestens 15 und besonders bevorzugt mindestens 25 Gew-% beträgt.

Ferner kann vorgesehen sein, dass die oxidative Veresterungsreaktion gemäß Schritt B) vorzugsweise mit einem molaren Verhältnis von Methanol zu Methacrolein im Bereich von 1:1 bis 50:1, besonders bevorzugt 1,5:1 bis 25:1 und speziell bevorzugt 2:1 bis 10:1 erfolgt.

Die Menge des einzusetzenden Katalysators variiert in Abhängigkeit von der Zusammensetzung des Einspeisungsgemisches und des Katalysators, der Reaktionsbedingungen, der Reaktionsarten und dergleichen. Wenn der Katalysator in Form einer Aufschlämmung eingesetzt wird, ist es bevorzugt, den Katalysator in einer Menge von 0,01 bis 0,5 kg/l der Reaktionssystemlösung einzusetzen.

Die oxidative Veresterungsreaktion kann auf jede herkömmliche Reaktionsart durchgeführt werden, wie durch eine Flüssigphasenreaktion oder Rieselbettreaktion. Beispielsweise kann jeder bekannte Reaktor, wie z.B. ein Blasensäulenreaktor, ein Röhrenreaktor mit Luftströmung oder ein Rührreaktor eingesetzt werden.

Der Druck, bei der diese Umsetzung durchgeführt wird, kann über einen breiten Bereich variiert werden. Überraschende Vorteile können durch einen Reaktionsdruck im Bereich von 2 bis 100 bar, bevorzugt 3 bis 80 bar, mehr bevorzugt 4 bis 50 bar und besonders bevorzugt 5 bis 20 bar erzielt werden.

Es ist bevorzugt, das Reaktionssystem durch Zugeben mindestens einer basischen Verbindung, die vorzugsweise aus der aus einer Alkalimetallverbindung und/oder einer Erdalkalimetallverbindung bestehenden Gruppe ausgewählt ist, wie ein Oxid, Hydroxid, Carbonat, Carboxylat und dergleichen, bei einem pH von 5 bis 9, besonders bevorzugt 6,5 bis 8 zu halten.

Die oxidative Veresterungsreaktion gemäß Schritt B) kann bei einer Temperatur im Bereich von vorzugsweise 10 °C bis 200 °C, besonders bevorzugt 40 bis 150 °C und speziell bevorzugt 60 bis 120 °C erfolgen.

Die Reaktionszeit oder Verweilzeit variiert in Abhängigkeit von anderen Reaktionsbedingungen; sie liegt jedoch bevorzugt im Bereich von 10 Minuten bis 48 Stunden, vorzugsweise 30 Minuten bis 24 Stunden und besonders bevorzugt 45 Minuten bis 2 Stunden.

Weitere Hinweise zur Durchführung einer oxidativen Veresterungsreaktion gemäß Schritt B) zur Synthese von MMA finden sich unter anderem in US 4,249,019 oder DE 3018071A1.

Durch die oxidative Veresterung unter den vorstehend genannten Bedingungen wird ein Reaktionsgemisch, das MMA als Hauptreaktionsprodukt enthält, erhalten. Zusätzlich zu MMA enthält das erhaltene Reaktionsgemisch auch nicht umgesetztes Methacrolein und nicht umgesetztes Methanol und geringe Mengen an Wasser und Methacrylsäure als Nebenprodukte. Das Reaktionsgemisch enthält außerdem Spuren von anderen Nebenprodukten, die Dimethacrolein und dergleichen umfassen.

Das in Schritt B) erhaltene Reaktionsprodukt kann auf bekannte Weise aufgearbeitet werden, um reines MMA zu erhalten. So kann das umgesetzte Reaktionsgemisch, das durch die oxidative Veresterung gemäß Schritt B) erhalten wird, zunächst durch Destillation aufgearbeitet werden.

Gemäß einer bevorzugten Ausführungsform kann das Reaktionsgemisch in einen Destillationsturm eingeleitet werden, wobei es bevorzugt in den mittleren Teil desselben eingeleitet wird: Im Allgemeinen kann ein azeotropes Gemisch von Methacrolein und Methanol über Kopf abdestilliert werden.

Aus dem Sumpf des Destillationsturms wird ein Gemisch erhalten, das flüssiges MMA, Methanol, Wasser und andere Nebenprodukte enthält. Dieses flüssige Gemisch wird durch ein herkömmliches Verfahren gereinigt. Im Allgemeinen können in dieser Aufreinigungseinheit mindestens eine, vorzugsweise zwei oder mehr Destillen zur Abtrennung von hochsiedenden und niedrigsiedenden Verbindungen enthalten sein.

In der vorliegenden Erfindung gibt es im Hinblick auf die Art des Destillationsturms, der vorzugsweise eingesetzt wird, um aus der durch die oxidative Veresterung erhaltenen Reaktionsmischung aufzuarbeiten, keine besondere Einschränkung, und jeder beliebige herkömmliche Destillationsturm, wie eine Bodenkolonne oder eine gepackte Kolonne, kann eingesetzt werden.

Da jedoch Methacrolein, MMA und Methacrylsäure, die in den Destillationsturm eingeleitet werden, leicht polymerisierbare Verbindungen sind, ist es bevorzugt, eine Destille mit einer Struktur einzusetzen, bei der ein Zusetzen mit Polymerisationsprodukten nicht auftritt und/oder die Polymerisationsprodukte leicht entfernt werden können. Spezifische Beispiele von Destillationstürmen umfassen Bodenkolonnen, die mit einem Siebboden, Kaskadenboden, Turbogridboden, Riffelboden oder dergleichen ausgestattet ist, und gepackte Kolonnen, die mit Packungsmaterialien regelmäßig (wie z.B. Mellapak von Sulzer) oder unregelmäßig (wie z.B. Raschig Superring von Raschig) gepackt ist.

In dem erfindungsgemäßen Verfahren variiert die geeignete Destillationstemperatur in dem Destillationsturm, der vorzugsweise eingesetzt wird, um aus der durch die oxidative Veresterung erhaltenen Reaktionsmischung aufzuarbeiten, in Abhängigkeit von dem Destillationsdruck, der Zusammensetzung der Flüssigkeit in dem Destillationsturm, der Anzahl der Böden in dem Destillationsturm und dergleichen. Um jedoch die Bildung der vorstehend genannten Polymerisationsprodukte und die Bildung von hochsiedenden Verbindungen, die einem Ausbeuteverlust an Methacrolein oder MMA darstellen, auf ein Minimum zu beschränken, ist es bevorzugt, dass die Destillationstemperatur so gering wie möglich ist. Wenn die Destillationstemperatur jedoch sehr gering gewählt wird, können Nachteile auftreten. Zu diesen gehören beispielsweise, dass der Destillationsdruck auch gering gewählt werden muss. Hierdurch kann es erforderlich sein, den Destillationsturm in einer unvorteilhaft großen Größe einzusetzen. Weiterhin kann der Einsatz eines Kühlmittels zum Konzentrieren der Gasphase bei dem obersten Tel des Destillationsturmes erforderlich werden. Vorzugsweise liegt die Destillationstemperatur oder die Temperatur der Flüssigkeit in der Kolonne im Bereich von 20 bis 100 °C, besonders bevorzugt von 40 bis 85 °C. Der Destillationsdruck ergibt sich aus dieser Temperatur,

Wie vorstehend erwähnt, können Methacrolein, MMA und gegebenenfalls weitere polymerisierbare Nebenprodukte, beispielsweise Methacrylsäure, in einen Destillationsturm eingeleitet werden, um das Reaktionsprodukt der oxidativen Veresterung in Methanol/Methacrolein- und MMA/Wasser-Gemische zu trennen.

Auf Grund der Polymerisierbarkeit ist es bevorzugt, dass ein oder mehrere Polymerisationsinhibitoren dem Prozess zugegeben werden. Polymerisationsinhibitoren, wie beispielsweise Hydrochinone, Hydrochinonether, wie Hydrochinonmonomethylether oder Di-tert-butylbrenzcatechin, Phenothiazin, N,N'-(Diphenyl)-p-phenylendiamin, 4 Hydroxy-2,2,6,6-tetramethylpiperidin-1-oxyl, p-Phenylendiamin, Methylenblau oder sterisch gehinderte Phenole, sind in der Fachwelt weithin bekannt. Diese Verbindungen können einzeln oder in Form von Mischungen eingesetzt werden und sind im Allgemeinen kommerziell erhältlich. Die Wirkung der Stabilisatoren besteht meist darin, dass sie als Radikalfänger für die bei der Polymerisation auftretenden freien Radikale wirken. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

Vorzugsweise werden insbesondere Phenole als Polymerisationsinhibitor eingesetzt. Besonders überraschende Vorteile können bei Verwendung von Hydrochinonmonomethylether erzielt werden. Bezogen auf das Gewicht der gesamten Zusammensetzung kann der Anteil der Inhibitoren einzeln oder als Mischung im Allgemeinen 0,001 - 0,5 Gew-%.

Vorzugsweise werden die Schritte A) und B) in einem kontinuierlichen Verfahren durchgeführt. Hierbei werden über einen beliebigen Zeitraum dauerhaft und stets Edukte in die Anlage zur Durchführung eines Verfahrens gemäß der vorliegenden Erfindung zugeführt und Produkte aus der Anlage entnommen. Dieser Zeitraum kann jedoch für Wartungs- und Instandsetzungsaufgaben unterbrochen werden.

Ferner kann vorgesehen sein, dass das Reaktorvolumen in Schritt A) kleiner ist als das Reaktorvolumen in Schritt B). Das Reaktorvolumen bezieht sich hierbei auf die Volumina in Schritt A) und Schritt B), in denen die eingesetzten Edukte in flüssiger Phase unter dem erhöhten Druck der jeweiligen Reaktion in die Produkte umgesetzt werden.

Vorteilhafterweise liegt das Verhältnis von Reaktorvolumen in Schritt A) zu Reaktorvolumen in Schritt B) im Bereich von 1:1000 bis 1:100, bevorzugt im Bereich von 1:800 bis 1:200 und besonders bevorzugt im Bereich von 1:500 bis 1:300

Typische Reaktorvolumina einer kontinuierlich betriebenen Produktionsanlage können zum Beispiel für Schritt A) ein Rohr(bündel)reaktor mit 0.1 bis 0.5 m$^3$ und für Schritt B) ein Rohr(bündel)reaktor mit 10 bis 15 m$^3$ oder ein kontinuierlich betriebener Rührkessel mit 50 bis 100 m$^3$ Inhalt sein, ohne dass diese Angaben eine Einschränkung bedeuten sollen.

Überraschend gelingt es, durch das vorliegende Verfahren im Vergleich zu konventionellen Verfahren, bei denen C4-Bausteine, beispielsweise Isobutylen oxidiert werden, das zu komprimierende Volumen, insbesondere an Gas deutlich zu verringern.

Bei dem erfindungsgemäßen Verfahren, bestehend aus der Kombination der Reaktionsschritte A) und B) ist es bei keinem der einzelnen Reaktionsschritte zwingend erforderlich, zusätzliches, d.h. nicht ohnehin in den Reaktanden bereits enthaltenes Wasser dem Reaktionsgemisch zuzuführen. Dies ist ein entscheidender Vorteil gegenüber dem Stand der Technik, da zusätzlich zugeführtes Wasser die Reaktionsströme und somit auch die benötigten Apparaturen vergrößert und in der Regel von den gewünschten Endprodukten auch wieder abgetrennt werden muss, was zusätzlichen energetischen Aufwand und Kosten bedeutet. Somit können bei dem erfindungsgemäßen Verfahren die Reaktionsvolumina und Ströme insgesamt gering gehalten werden.

Das Verfahren der Asahi gemäß US 5,969,178 und US 7,012,039 benötigt beispielsweise während der Gasphasenoxidation der C$_4$-Komponente die Zugabe und anschließende Wiederabtrennung überstöchiometrischer Mengen an Wasser. Auch die bisher bekannten Verfahren, die Methacrolein ausgehend von Ethylen über Propionaldehyd herstellen, dieses dann zu Methacrylsäure oxidieren und zu MMA weiter verestern, benötigen bei der Gasphasenoxidation des Methacroleins zu Methacrylsäure die Zugabe überstöchiometrischer Mengen an Wasser, um die Aktivität des Oxidationskatalysators dauerhaft zu gewährleisten.

Bei dem erfindungsgemäßen Verfahren, bestehend aus der Kombination der Reaktionsschritte A) und B), ist daher die Gesamtmenge des während der Reaktionsdurchführung separat zugegebenen Wassers nicht größer als 100 Mol-%, bevorzugt 50 Mol-%, besonders bevorzugt 30 Mol-% und noch mehr bevorzugt nicht größer als 10 Mol-%, jeweils bezogen auf Methacrolein. In einer besonders vorteilhaften Ausführungsform des erfindungsgemäßen Verfahrens wird in keinem der Reaktionsschritte A) und B) während der Reaktionsdurchführung Wasser separat zur Reaktionsmischung zugegeben. Reaktionswasser und Wasserzugabe für Aufarbeitungsschritte sind hiervon jeweils ausgenommen.

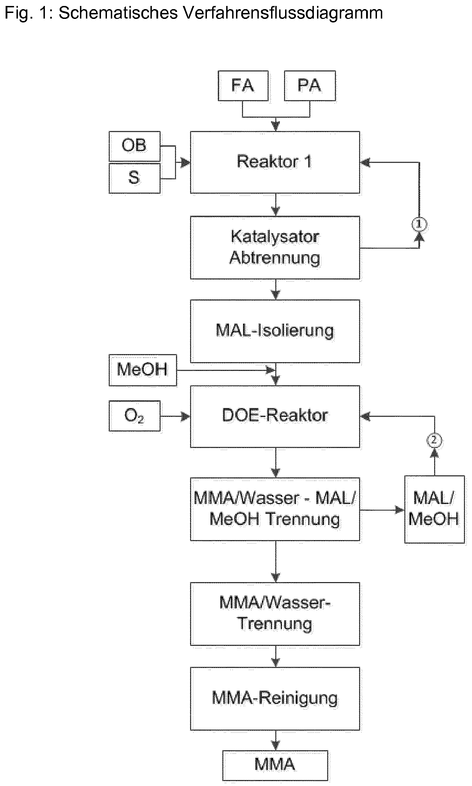

Fig. 1 zeigt beispielhaft eine schematische Darstellung des erfindungsgemäßen Verfahrens, ohne dass hierdurch eine Begrenzung der Erfindung erfolgen soll.

Formaldehyd (FA) und Propanal (PA) werden vorgemischt oder einzeln dem Reaktor 1 zugeführt, ebenso vorgemischt oder einzeln die organische Base (OB) und der Säurekatalysator (S). Nach erfolgter Adlolkondensation und der Katalysatorabtrennung wird das Methacrolein (MAL) isoliert. Der Katalysator kann über Strom (1) in den Reaktor 1 zurückgeführt werden. Das MAL und Methanol (MeOH) werden dem oxidativen Veresterungsreaktor (DOE-Reaktor) zugeführt. Dieser wird mit einem sauerstoffhaltigen Gas ($O_2$) gespeist. Aus dem Veresterungsreaktor wird das nicht umgesetzte MAL als MAL/Methanol-Azeotrop in einer MMA/Wasser-MAL/MeOH-Trennung abgetrennt und über Strom (2) in den DOE-Reaktor zurückgeführt. Danach erfolgt eine MMA/Wasser-Trennung und weitere Reinigung des Roh-MMA.

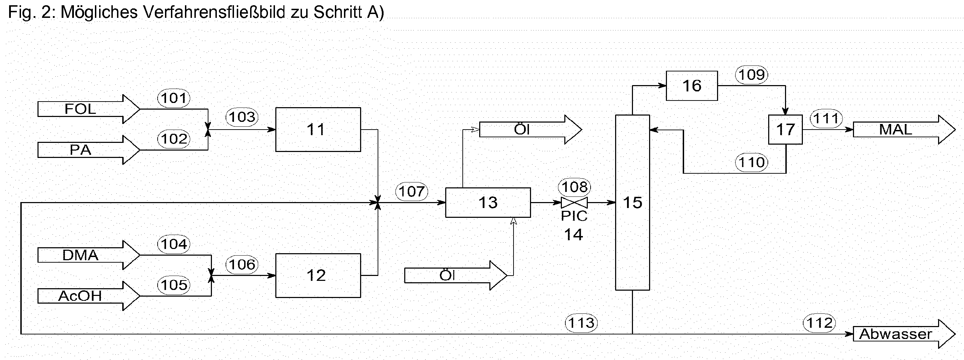

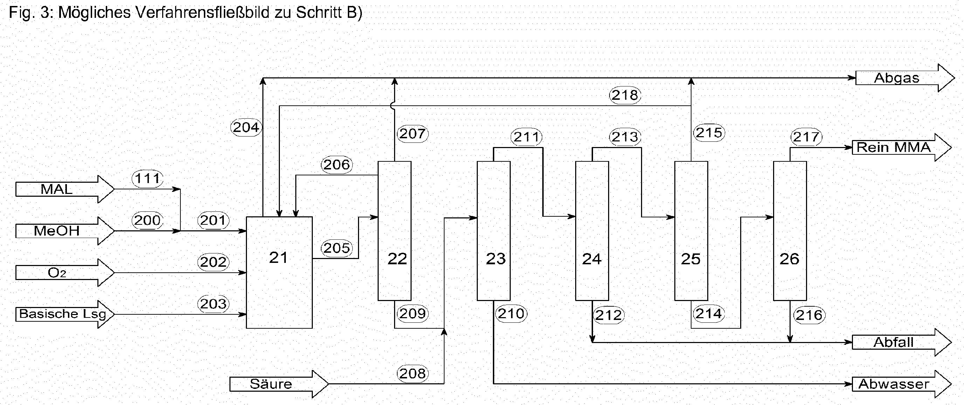

In Fig. 2 ist eine mögliche Anlage zur Umsetzung von Formaldehyd mit Propanal zu Methacrolein (Schritt A) gezeigt. Wässriges Formalin (101) wird mit Propionaldehyd (102) gemischt und als Strom (103) in den Vorwärmer (11) geführt. Dimethylamin (40%ige wässrige Lösung) (104) und Essigsäure (105) werden gemischt und als Strom (106) in den Vorwärmer (12) geführt. Das Betreiben der Vorwärmer (11) und (12) ist optional. Die Mischung aus den Abläufen von (11) und (12) wird dem Rohrreaktor (13) als Strom (107) zugeführt. Der Rohrreaktor (13) wird mittels eines Ölbades auf die Reaktionstemperatur aufgeheizt. Nach dem Rohrreaktor wird das Gemisch (108) in Ventil (14) entspannt und der Kolonne (15) zugeführt. Der Sumpfablauf der Kolonne wird geteilt (50/50), ein Teil zum Strom (107) in den Reaktor (13) zurückgeführt, der andere als Abwasserstrom (112) entsorgt. Der im Kopf der Kolonne erhaltene Strom wird im Kondensator (16) verflüssigt und als Strom (109) dem Phasentrenner (17) zugeführt. In diesem wird eine methacroleinreiche Phase (111) als Produkt in den Anlagenteil "direkte oxidative Veresterung" gemäß Fig. 3 abgeführt, wobei optional eine Trocknung des Stroms (111) durch azeotrope Destillation erfolgen kann (nicht gezeigt). Der wässrige Ablauf des Phasentrenners (17) wird als Strom (110) in die Kolonne (15) zurückgeführt.

In Fig. 3 ist eine mögliche Vorrichtung zur Erzeugung von MMA aus MAL dargestellt, die zur Durchführung der direkten oxidativen Veresterung (Schritt B) geeignet ist. Methanol wird über Zuleitung (200) der Leitung (111) zugeführt, über die Methacrolein aus dem Schritt A) des Prozesses entnommen wird. Über die Zuleitung (202) wird Luft (oder sauerstoffhaltiges Gasgemisch) in den Reaktor (21), der einen für die direkte oxidative Veresterung geeigneten Katalysator enthält, eingeleitet, sowie über die Zuleitung (203) eine basische Zusammensetzung, die vorzugsweise Methanol und NaOH umfasst, zur Einstellung des pH-Werts. Die Hilfsaggregate, wie Pumpen, Heizelemente, Wärmetauscher und Kondensatoren sind in Fig. 3 nicht dargestellt. Es können auch gegebenenfalls mehrere in Serie geschaltete Reaktoren (21) verwendet werden (nicht gezeigt).

Aus dem Reaktor (21) werden Abgase durch die Abgaswascheinrichtung über Leitung (204) abgeleitet, wobei Methanol, MMA und Methacrolein zumindest teilweise in einem oder mehreren Kondensatoren kondensiert und in den Reaktor (21) zurückgeführt werden können (nicht gezeigt).

Die im Reaktor (21) erhaltene Reaktionsmischung wird über Leitung (205) in die Destillationskolonne (22) eingeleitet, wobei Methacrolein (oder methacroleinhaltiges Gemisch) über Leitung (206) zurück in den Reaktor (21) geführt wird. Gase und weitere leichtsiedende Komponenten können über Kopf der Destillationskolonne (22) aus dem Reaktionsgemisch abgetrennt und über Leitung (207) dem Abgas zugeführt werden. Die aus dem Sumpf der Destillationskolonne (22) über Leitung (209) entnommene Zusammensetzung umfasst im Wesentlichen MMA, welches Methanol, Methacrylsäure, Natriummethacrylat und andere Komponenten enthalten kann.

Über Zuleitung (208) kann aus einem Vorratsbehälter eine Säure oder säurehaltiges Gemisch, beispielsweise wässrige Schwefelsäure der Leitung (209) zugeleitet werden. Die so erhaltene Mischung wird in eine Wasser/Öl Trennungsanlage (23) geführt, die beispielsweise eine Zentrifuge enthalten kann, und in wässrige und organische Phase getrennt. An dieser Stelle können mehrere analoge Wasser/Öl Trennungsanlagen parallel geschaltet werden, so dass man diese bei Bedarf abwechselnd betreiben kann (nicht gezeigt). Die wässrige Phase der Trennungsanlage (23) kann über Leitung (210) einer Abwasseraufbereitung zugeführt werden, während die organische Phase über Leitung (211) der Destillationskolonne (24) zur Abtrennung der hochsiedenden Komponenten zugeführt wird. Aus dem Sumpf dieser Kolonne (24) können über Leitung (212) hochsiedende Komponenten, z. B. Methacrylsäure, zur weiteren Nachbehandlung entnommen werden. Roh-MMA wird aus dem Kopf der Kolonne (24) über Leitung (213) entnommen und der Kolonne (25) zugeführt. Die leichtsiedenden Komponenten (wie z.B. Methanol und Methacrolein) können aus dem Kopf dieser Kolonne über Leitung (215) entnommen und über Leitung (218) zurück in den Reaktor (21) geführt werden, ein Teil davon kann ausgeschleust und dem Abgasstrom (204) zugeführt werden. Aus dem Sumpf der Kolonne (25) kann über Leitung (214) aufgereinigtes MMA entnommen werden und der letzten Kolonne für die MMA Reinigung (26) zugeführt werden. Rein-MMA wird aus dem Kopf der Kolonne über Leitung (217) entnommen, während die restlichen schwersiedenden Komponenten aus dem Sumpf der Kolonne über Leitung (216) zur weiteren Nachbehandlung oder zur Rückführung vor Kolonne (24) (nicht gezeigt) entnommen werden können.

**Bezugszeichenliste**

**Fig.1**

| OB | organische Base |
|----|----|
| S | Säure |
| MeOH | Methanol |
| $O_2$ | sauerstoffhaltiges Gas |
| MMA | Methylmethacrylat |
| FA | Formalin (wässrige Formaldehyd-Lösung) |
| PA | Propanal |
| MAL | Methacrolein |
| DOE | direkte oxidative Veresterung |
| ① | Katalysator-Rückführung |
| ② | Methacrolein/Methanol-Rückführung |

**Fig. 2**

| FOL | Formalin (wässrige Formaldehyd-Lösung) |
|----|----|
| PA | Propanal |
| DMA | wässrige Dimethylamin-Lösung |
| AcOH | Essigsäure |
| MAL | Methacrolein |
| 11 | Wärmetauscher (Vorwämer) |
| 12 | Wärmetauscher (Vorwämer) |
| 13 | Reaktor (Rohrreaktor) |
| 14 | Druckhalteventil |
| 15 | MAL-Destillationskolonne |
| 16 | Kondensator |
| 17 | Phasentrenner |
| 101 | Leitung wässriges Formalin |
| 102 | Leitung Propanal |
| 103 | Leitung in Wärmetauscher |
| 104 | Leitung Dimethylamin (40%ige wässrige Lösung) |
| 105 | Leitung Essigsäure |
| 106 | Leitung in Wärmetauscher |
| 107 | Leitung in Reaktor |
| 108 | Leitung Produktgemisch zur Kolonne |
| 109 | Leitung Kondensat |
| 110 | Rückstrom in Kolonne |
| 111 | MAL zu Stufe B) |
| 112 | Leitung zu Abwasser |
| 113 | Rückführung Sumpfablauf |

**Fi. 3**

| MAL | Methacrolein |
|----|----|
| MeOH | Methanol |
| $O_2$ | sauerstoffhaltiges Gas |
| MMA | Methylmethacrylat |

| | |
|---|---|
| 21 | Reaktor |
| 22 | MAL Abtrennung |
| 23 | Wasser/Öl Trennung |
| 24 | Abtrennung Hochsieder |
| 25 | Abtrennung Leichtsieder |
| 26 | MMA-Feinreinigung |
| 111 | MAL aus Stufe A) |
| 200 | Zuleitung Methanol |
| 201 | Zuleitung MAL/Methanol |
| 202 | Zuleitung sauerstoffhaltiges Gas |
| 203 | Zuleitung basische Zusammensetzung |
| 204 | Abgasstrom |
| 205 | Produktstrom zur Kolonne 22 |
| 206 | Rückführung MAL |
| 207 | Abgasstrom |
| 208 | Zuleitung Säure |
| 209 | Produktstrom zur Wasser/Öl-Trennung |
| 210 | Ableitung wässrige Phase |
| 211 | Produktstrom zur Kolonne 24 |
| 212 | Ableitung Hochsieder |
| 213 | Produktstrom zur Kolonne 25 |
| 214 | Produktstrom zur Kolonne 26 |
| 215 | Ableitung Leichtsieder |
| 216 | Sumpfablauf |
| 217 | Leitung Rein-MMA |
| 218 | Rückführung Leichtsieder |

Die nachfolgenden Beispiele dienen zur näheren Erläuterung bevorzugter Ausführungsformen der vorliegenden Erfindung, ohne dass hierdurch eine Begrenzung der Erfindung erfolgen soll.

Beispiel 1

In einer Anlage entsprechend Fig: 2 wird kontinuierlich Propanal (PA) mit Formaldehyd unter Verwendung von Dimethylamin (DMA) und Essigsäure (AcOH) umgesetzt. 251 g/h PA und 349 g/h einer 37-prozentigen Formalinlösung werden homogen vorgemischt (Verhältnis molar 1:1). 18,7 g/h einer Katalysatorlösung mit 24,8 % Dimethylamin und 37,9 % Essigsäure werden in den Vorheizer 12 gefahren. Beide Ströme werden vor Vereinigung auf eine Temperatur von 170°C erhitzt. Die vorerhitzten Ströme werden in einem T-Mischer vereinigt, der direkt mit einem Strömungsrohrreaktor (1/16 Zoll Rohr mit einer Länge von 4,2 m) verbunden ist. Die Thermostatisierung des Reaktors erfolgt mit einem Ölbad, das bei 180°C betrieben wird, die Verweilzeit beträgt 10 s, der Druck im Rohrreaktor 70 bar. Nach dem Rohrreaktor wird das Gemisch in Ventil (14) entspannt und der Kolonne (15) zugeführt. 335 g/h des Sumpfablaufes werden in den Reaktor (13) zurückgeführt, 370 g/h des Sumpfablaufes als Abwasser entsorgt. Nach Verflüssigung des Kopfstroms im Kondensator (16) und Phasentrennung in (17) wird eine methacroleinreiche Phase mit einem Methacrolein-Gehalt von 96,5 % als Produkt (111) abgeführt und der wässrige Ablauf des Phasentrenners in die Kolonne (15) zurückgeführt. Der Umsatz beträgt 99,9%, die Ausbeute 98,1 %, bezogen auf Propionaldehyd. Das in den Beispielen 2 bis 4 eingesetzte Methacrolein hatte einen Restwassergehalt von 1,7 Gew%.

Beispiel 2

Katalysator 1 (0,9%Au-1,1%NiO auf $SiO_2$-$Al_2O_3$-MgO) wurde analog Beispiel 1 der EP 2 210 664 A1 hergestellt. Eine Lösung von 375 g Aluminiumnitrat-Nonahydrat, 256 g Magnesiumnitrat-Hexahydrat und 54 g 60 % Salpetersäure in 500 mL Wasser wurden bei 15 °C zu 2 kg Silica Sol Lösung mit 10-20 nm Partikelgröße, (Nissan Chemical Industries, Snowdex N-30, 30 wt% $SiO_2$) zugetropft. Das Gemisch wurde 24 Stunden bei 50 °C gerührt, danach auf Raumtemperatur abgekühlt, sprühgetrocknet (130 °C) und kalziniert (300 - 600 °C, insgesamt 10 Stunden). 30 g dieses $SiO_2$-$Al_2O_3$-MgO Trägers wurden in 100 mL Wasser suspendiert und auf 90 °C aufgeheizt. Diese Suspension wurde nach 15 Min bei 90 °C zu einer Lösung von 1,64 g Nickelnitrat-Hexahydrat und 530 mg Goldsäure (HAuCl4) in 100 mL Wasser zugegeben. Nach weiteren 30 Min Rühren bei 90 °C wurde abgekühlt und der Feststoff abgetrennt, anschließend noch dreimal mit 100 mL frischem Wasser jeweils 5 Minuten bei 20 °C gerührt und abfiltriert. Der Katalysator wurde bei 105 °C innerhalb von 10 Stunden getrocknet und bei 450 °C innerhalb von 5 Stunden an der Luft kalziniert. Das so erhaltene violette Pulver enthielte nach ICP Analyse 1,1% Ni und 0,9% Au. Die mittlere Partikelgröße von Gold Nanopartikeln (TEM) betrug weniger als 5 nm.

Eine Mischung aus 0,67 g Methacrolein (aus Beispiel 1), 5,65 g Methanol und 504 mg Au-Katalysator 1 wurde in einem Autoklav unter 11 bar eines $O_2/N_2$ Gasgemischs (7 Vol-% $O_2$) bei 80 °C innerhalb von 2 Stunden gerührt, anschließend abgekühlt, filtriert und mittels GC analysiert. Der Umsatz von MAL betrug 98,4%, die Ausbeute an MMA 94,8%, die Selektivität zu MMA 96,3%, Raum-Zeit-Ausbeute 9,3 mol MMA/kg Kat-h.

Beispiel 3

Katalysator 2 (1%Au-5%ZnO-5%MgO auf $SiO_2$) wurde analog dem Beispiel 1-6 der EP1393800A1 hergestellt. 89 g eines kommerziell zugänglichen Si02-Trägers (Cariact Q-10, 75-150$\mu$m, Fuji Silisia) wurde mit einer Lösung von 18,3 g Zinknitrat Hexahydrat und 12,8 g Magnesiumnitrat Hexahydrat in 90 mL Wasser imprägniert, bei 120 °C innerhalb von 12 Stunden getrocknet und dann bei 600 °C innerhalb von 4 Stunden kalziniert. In 300 mL einer 20 mmol/L Lösung HAuCl4 wurde mit einer 0,5 M Lösung NaOH bei 70 °C pH = 7 eingestellt und bei dieser Temperatur der zuvor hergestellte $SiO_2$-ZnO-MgO Träger unter Rühren zugegeben. Nach weiterem Rühren für eine Stunde bei 70 °C wurde abgekühlt, filtriert und der Katalysator noch dreimal mit 400 mL frischem Wasser jeweils 5 Minuten bei 20 °C gerührt. Nach 10 Stunden Trocknung bei 100 °C wurde innerhalb von 3 Stunden bei 400 °C an der Luft kalziniert. Das erhaltene violette Pulver enthielt nach ICP Analyse 1,5% Au. Die mittlere Partikelgröße von Gold Nanopartikeln betrug weniger als 5 nm.

Eine Mischung aus 0,60 g Methacrolein (aus Beispiel 1), 5,76 g Methanol und 300 mg Katalysator 2 wurde in einem Autoklav unter 11 bar eines $O_2/N_2$ Gasgemischs (7 Vol-% $O_2$) bei 80 °C innerhalb von 2 Stunden gerührt, anschließend abgekühlt, filtriert und mittels GC analysiert. Der Umsatz von MAL betrug 85,5%, die Ausbeute an MMA 83,4%, die Selektivität zu MMA 97,5%, Raum-Zeit-Ausbeute 14,0 mol MMA/kg Kat-h.

Beispiel 4

Katalysator 3 (1,5%Au-5%$La_2O_3$-5%MgO auf $SiO_2$) wurde analog dem Beispiel 1-7 der EP1393800A1 hergestellt. 88,5 g eines kommerziell zugänglichen Si02-Trägers (Cariact Q-10, 75-150$\mu$m, Fuji Silisia) wurde mit einer Lösung von 13,3 g Lanthannitrat-Hexahydrat und 12,8 g Magnesiumnitrat-Hexahydrat in 90 mL Wasser imprägniert, anschließend bei 120 °C innerhalb von 12 Stunden getrocknet und dann bei 600 °C innerhalb von 4 Stunden kalziniert. In 450 mL einer 20 mmol/L Lösung HAuCL4 wurde bei 70 °C mit einer 0.5 M Lösung NaOH pH = 7 eingestellt und bei dieser Temperatur der zuvor hergestellte $SiO_2$-$La_2O_3$ Träger unter Rühren zugegeben. Nach weiterem Rühren bei 70 °C für eine Stunde wurde abgekühlt, filtriert und der Katalysator noch dreimal mit 400 mL frischem Wasser jeweils 5 Minuten bei 20 °C gerührt. Nach 10 Stunden Trocknung bei 100 °C wurde bei 400 °C innerhalb von 3 Stunden an der Luft kalziniert. Das erhaltene violette Pulver enthielt nach ICP Analyse 1,5% Au. Die mittlere Partikelgröße von Gold-Nanopartikeln betrug weniger als 5 nm.

Eine 42,9 %-ige Lösung von Methacrolein (aus Beispiel 1) in Methanol wurde mit einer Flowrate von 420 g/h kontinuierlich einem mechanisch gerührten 2,5 L Rührkesselreaktor mit einem Katalysatorseparator zugeführt, in dem 255 g von Katalysator 3 vorgelegt wurden. Der pH des Reaktionsgemisches wurde mittels Zugabe einer NaOH Lösung in Methanol (1-4 Gew.-%) mit einer Flowrate von 40 g/h bei ca. 7 gehalten. Dem Reaktor wurde bei 5 bar und 80 °C kontinuierlich so viel Luft zugeführt, dass der Restanteil an Sauerstoff im Abgas ca. 4 Vol-% $O_2$ betrug. Das kontinuierlich entnommene Produktgemisch wurde mittels GC analysiert. 50 h nach dem Start des Prozesses betrug der Umsatz von Methacrolein 78,5 %, die Ausbeute an MMA 76,5 %, die Selektivität zu MMA 97,4%, Raum-Zeit-Ausbeute 9,8 mol MMA/kg Kat-h. Die im Reaktor (21) erhaltene Reaktionsmischung wurde in die Destillationskolonne (22) (45 Stufen, Durchmesser 15 cm, Höhe 6 m) an der 30. Stufe (von oben) eingeleitet. Die Sumpftemperatur betrug 84 °C, die Kopftemperatur 31 °C. Das Methacrolein/Methanol Gemisch wurde an der Stufe 5 (von oben) entnommen und zurück in den Reaktor (21) geführt. Die aus dem Sumpf der Destillationskolonne (22) mit einer Flowrate von 500 g/h entnommene Zusammensetzung bestand aus MMA, welches zusätzlich Methanol, Methacrylsäure, Natriummethacrylat und andere Komponenten enthielt. Aus einem Vorratsbehälter wurde diesem Strom (Leitung 209) kontinuierlich so viel einer 10 %-igen wässrigen Schwefelsäure zugeleitet, dass der pH des resultierenden Gemisches 2 betrug. Die so erhaltene Mischung wurde in der Wasser/Öl Trennungsanlage(23) mittels einer Zentrifuge getrennt. Die organische Phase wurde mit einer Flowrate von 375 g/h der Destillationskolonne (24) (30 Stufen, Durchmesser 10 cm, Höhe 5 m) an der 20. Stufe (von oben) zugeführt. Diese Kolonne wurde bei 150 mmHg betrieben, wobei die Sumpftemperatur 70 und die Kopftemperatur 45 °C betrug. Rohes MMA wurde mit einer Flowrate von 288 g/h aus dem Kopf der Kolonne (24) entnommen und der Kolonne (25) (30 Stufen, Durchmesser 10 cm, Höhe 5 m) an der 10. Stufe (von oben) zugeführt. Die Kolonne (25) wurde bei 250 mmHg betrieben, wobei die Sumpftemperatur 80 und die Kopftemperatur 50 °C betrug. Aus dem Sumpf der Kolonne (25) wurde aufgereinigtes MMA mit einer Flowrate von 281 g/h entnommen und der Kolonne (26) (70 Stufen, Durchmesser 10 cm, Höhe 5 m) an der 35. Stufe (von oben) zugeführt. Die Kolonne (26) wurde bei 140 mmHg betrieben, wobei die Sumpftemperatur 80 und die Kopftemperatur 55 °C betrug. Reines MMA wurde mit einer Flowrate von 250 g/h aus dem Kopf der Kolonne (26) entnommen. Die Gesamtausbeute an isoliertem Methylmethacrylat aus Methacrolein betrug nach der oben beschrieben Aufarbeitung 97,2%.

Tab. 1: Zusammenfassung Reaktionen Schritte A) und B):

| Beispiel | Reaktion | Ausbeute | Selektivität | Raum-Zeit-Ausbeute Schritt B | Gesamtselektivität. PA zu MMA |
|---|---|---|---|---|---|
| | | | | [mol MMA/kg Kat-h] | |
| 1 | PA - MAL | 98,1 | 98,2 | | |
| 2 | MAL - MMA | 94,8 | 96,3 | 9,3 | 94,6 |
| 3 | MAL - MMA | 83,4 | 97,5 | 14,0 | 96,0 |
| 4 | MAL - MMA | 76,5 | 97,4 | 9,8 | 95,6 |

Vergleichswerte aus dem Stand der Technik:

In den folgenden Vergleichsbeispielen (Vergleich 5-8) sind mögliche Verfahren und Kombinationen aus verschiedenen Verfahren gemäß dem Stand der Technik mit den entsprechenden Selektivitäten der einzelnen Schritte und den Gesamtverfahren zusammengefasst.

Vergleich 5

Reaktion A: Isobutylen $\rightarrow$ Methacrolein

Reaktion B: Methacrolein $\rightarrow$ Methacrylsäure

Reaktion C: Methacrylsäure $\rightarrow$ Methylmethacrylat

U = Umsatz

S = Selektivität

Reaktion A (z.B. US7012039B2, Beispiel 1, Druck < 2 bar):

S(MAL) = 88,3%,

S(MAS) = 2,4%

Wasserzusatz $H_2O$/MAL = 1,7 (mol/mol)
Reaktion B, (z.B EP0376117B1, Beispiel 1, Druck < 2 bar)

S(MAS) = 89,2%

Wasserzusatz $H_2O$/MAL = 5,5 (mol/mol)

Reaktion C (z.B. US20020188151A1) S(MMA) ~ 100%

Gesamtselektivität der Reaktionen A + B + C = S(MAL+MAS aus IBN)*S(MAS aus MAL)*S(MMA aus MAL) = **80,9%**

Vergleich 6:

Reaktion A: Isobutylen → Methacrolein
Reaktion B: Methacrolein → Methylmethacrylat

Reaktion A (z.B. US7012039B2, Beispiel 1, Druck < 2 bar):
S(MAL) = 88,3%,
S(MAS) = 2,4%
Wasserzusatz $H_2O$/MAL = 1,7 (mol/mol)
Reaktion B, Variante1 (US7012039B2, Beispiel 1):
S(MMA) = 90,7%
Reaktion B, Variante 2 (EP 2 210 664 A1, Beispiel 7):
S (MMA) = 97,2%

Gesamtselektivität A + B (Variante 1): S(MMA aus IBN) = S(MAL)*S(MMA) = 80,1%
Gesamtselektivität A + B (Variante 2): S(MMA aus IBN) = S(MAL)*S(MMA) = 85,8%

Vergleich 7

Reaktion A: Propionaldehyd→ Methacrolein
Reaktion B: Methacrolein → Methacrylsäure
Reaktion C: Methacrylsäure → Methylmethacrylat

Reaktion A (z.B. DE3213681A1, Beispiel 1)
S(MAL) = 98,1 %

Reaktion B (z.B. EP0376117B1, Beispiel 1, Druck < 2 bar)
S(MAS) = 89,2%

Wasserzusatz MAL/$H_2O$ = 5,5 (mol/mol)

Reaktion C (z.B. US20020188151A1) S(MMA) ~ 100%

Gesamtselektivität der Schritte A + B + C = S(MMA aus PA) = S(MAL aus PA)*S(MAS aus MAL)*S(MMA aus MAS) = **87,5%**

Vergleich 8

Reaktion A: Propionaldehyd→ Methacrolein

Reaktion B: Methacrolein → Methylmethacrylat

Reaktion A, (CN101074192A1, Druck < 2 bar)
S(MAL) = 95,2%

Reaktion B, (CN101074192A1)
S(MMA) = 98,2%

Gesamtselektivität der Schritte A + B: S(MMA aus PA) = S(MAL)*S(MMA) = 93,5%

Tab. 2: Vergleich der Verfahren (Teil 1)

| Vergleich | Reaktion | Gesamtselektivität | H$_2$O-extra [mol/mol MMA] |
|---|---|---|---|
| 5 | IBN - MAL - MAS - MMA | 80,9 | 7,2 |
| 6 | IBN - MAL - MMA | 80,1 85,8 | 1,7 |
| 7 | PA-MAL-MAS-MMA | 87,5 | 5,5 |
| 8 | PA - MAL - MMA | 93,5 | - |
| 4 | PA - MAL - MMA | 95,6 | - |

Bei den Vergleichsverfahren 5 bis 7 werden neben deutlich schlechteren Gesamtselektivitäten insbesondere große molare Mengen an separat zugesetztem Wasser während der Reaktionsschritte benötigt.

Tab. 3: Vergleich der Verfahren (Teil 2)

| Beispiel/ Vergleich | Reaktion | Gesamtselektivität | Kat für MAL Synthese [mol Amin/ mol MAL] | Kat. Für MMA Synthese [Mol MMA/kg Kat-h] |
|---|---|---|---|---|
| 8 | PA - MAL - MMA | 93,5 | 1,040 | 3,7 |
| 4 | PA - MAL - MMA | 95,6 | 0,024 | 9,8 |

Wie aus der vorstehenden Tabelle ersichtlich ist, kann die Umsetzung gemäß der Erfindung (Beispiel 4) im Schritt A) mit katalytischen Mengen Aminbase bezogen auf MAL durchgeführt werden, während Vergleich 8 dazu eine überstöchiometrische Menge Katalysator benötigt. Die Raum-Zeit-Ausbeute von Schritt B) des erfindungsgemäßen Verfahrens ist mit einem Wert von 9,8 ebenfalls mehr als doppelt so hoch wie bei Vergleich 8.

**Patentansprüche**

1. Verfahren zur Herstellung von Methylmethacrylat, umfassend die Schritte:

   A) Herstellung von Methacrolein aus Propanal und Formaldehyd und
   B) Umsetzung des in Schritt A) erhaltenen Methacroleins in einer oxidativen Veresterungsreaktion zu Methylmethacrylat,

   **dadurch gekennzeichnet, dass** die beiden Schritte A) und B) in flüssiger Phase bei einem Druck von 2 bis 100 bar erfolgen und Schritt B) in Gegenwart eines heterogenen, Metalle und/oder Metalloxide umfassenden, edelmetallhaltigen Katalysators durchgeführt wird.

2. Verfahren gemäß Anspruch 1, **dadurch gekennzeichnet, dass** Schritt A) in Gegenwart von 0,1 bis 20 Mol-% organische Base und 0,1 bis 20 Mol-% Säure, jeweils bezogen auf Propanal, durchgeführt wird.

3. Verfahren gemäß Anspruch 1 oder 2, **dadurch gekennzeichnet, dass** Schritt A) bei einer Temperatur von 100 bis 300 °C durchgeführt wird.

4. Verfahren gemäß mindestens einem der vorhergehenden Ansprüche, **dadurch gekennzeichnet, dass** Schritt A) bei einem Druck von 5 bis 100 bar durchgeführt wird.

5. Verfahren gemäß mindestens einem der vorhergehenden Ansprüche, **dadurch gekennzeichnet, dass** der zur oxidativen Veresterungsreaktion gemäß Schritt B) eingesetzte heterogene Oxidationskatalysator eines oder mehrere ultrafein verteilte Metalle mit einer durchschnittlichen Teilchengröße von < 20 nm umfasst, welches ausgewählt ist aus der Gruppe bestehend aus Gold, Palladium, Ruthenium, Rhodium und Silber.

6. Verfahren gemäß mindestens einem der vorhergehenden Ansprüche, **dadurch gekennzeichnet, dass** der zur oxidativen Veresterungsreaktion gemäß Schritt B) eingesetzte heterogene Oxidationskatalysator eines oder mehrere Mitglieder der Gruppe umfasst, die aus Lithium, Natrium, Kalium, Calzium, Magnesium, Scandium, Yttrium, Lanthan und andere Lanthanoide mit den Ordnungszahlen 58 bis 71, Silizium, Titan, Zirkonium, Hafnium, Vanadium, Niobium, Tantal, Chrom, Molybdän, Wolfram, Mangan, Rhenium, Eisen, Ruthenium, Osmium, Kobalt, Rhodium, Iridium, Nickel, Palladium, Platin, Kupfer, Silber, Gold, Zink, Cadmium, Bor, Aluminium, Gallium, Indium, Thallium, Germanium, Zinn, Blei, Antimon, Bismut, Tellur, welche jeweils in metallischer und/oder oxidierter Form vorliegen, besteht.

7. Verfahren gemäß mindestens einem der Ansprüche 2 bis 6, **dadurch gekennzeichnet, dass** die Umsetzung von Propanal mit Formaldehyd zu Methacrolein gemäß Schritt A) unter Verwendung eines sekundären Amins als organische Base erfolgt.

8. Verfahren gemäß mindestens einem der Ansprüche 2 bis 7, **dadurch gekennzeichnet, dass** in Schritt A) mindestens eine organische Säure eingesetzt wird.

9. Verfahren gemäß mindestens einem der Ansprüche 2 bis 8, **dadurch gekennzeichnet, dass** das molare Verhältnis von Säure zu organischer Base im Bereich von 20:1 bis 1:20 liegt.

10. Verfahren gemäß mindestens einem der vorhergehenden Ansprüche, **dadurch gekennzeichnet, dass** die Umsetzung gemäß Schritt A) bei einer Verweilzeit im Bereich von 0,1 bis 300 Sekunden durchgeführt wird.

11. Verfahren gemäß mindestens einem der vorhergehenden Ansprüche, **dadurch gekennzeichnet, dass** die Umsetzung gemäß Schritt B) bei einem Druck im Bereich von 2 bis 50 bar durchgeführt wird.

12. Verfahren gemäß mindestens einem der vorhergehenden Ansprüche, **dadurch gekennzeichnet, dass** die oxidative Veresterungsreaktion gemäß Schritt B) bei einer Temperatur im Bereich von 10 bis 200 °C erfolgt.

13. Verfahren gemäß mindestens einem der vorhergehenden Ansprüche, **dadurch gekennzeichnet, dass** die oxidative Veresterungsreaktion gemäß Schritt B) mit einem molaren Verhältnis von Methanol zu Methacrolein im Bereich von 1:1 bis 50:1 erfolgt.

14. Verfahren gemäß mindestens einem der vorhergehenden Ansprüche, **dadurch gekennzeichnet, dass** die Schritte A) und B) in einem kontinuierlichen Verfahren durchgeführt werden.

15. Verfahren gemäß mindestens einem der vorhergehenden Ansprüche, **dadurch gekennzeichnet, dass** das Reaktorvolumen in Schritt A) kleiner ist als das Reaktorvolumen in Schritt B) und das Verhältnis der beiden Reaktorvolumina im Bereich von 1:1000 bis 1:100 liegt.

16. Verfahren gemäß mindestens einem der vorhergehenden Ansprüche, **dadurch gekennzeichnet, dass** die Gesamtmenge des in den beiden Schritten A) und B) während der Reaktionsdurchführung separat zugegebenen Wassers nicht größer ist als 100 Mol-% bezogen auf Methacrolein.

17. Verfahren gemäß mindestens einem der vorhergehenden Ansprüche, **dadurch gekennzeichnet, dass** in den beiden Schritten A) und B) während der Reaktionsdurchführung überhaupt kein Wasser separat zugegeben wird.

## Claims

1. Process for producing methyl methacrylate, comprising the following steps:

   A) producing methacrolein from propanal and formaldehyde and
   B) reacting the methacrolein obtained in step A) in an oxidative esterification reaction to give methyl methacrylate,

   **characterized in that** the two steps A) and B) take place in a liquid phase at a pressure of from 2 to 100 bar, and step B) is carried out in the presence of a heterogeneous noble-metal-containing catalyst comprising metals and/or comprising metal oxides.

2. Process according to Claim 1, **characterized in that** step A) is carried out in the presence of from 0.1 to 20 mol% of organic base and from 0.1 to 20 mol% of acid, based in each case on propanal.

3. Process according to Claim 1 or 2, **characterized in that** step A) is carried out at a temperature of from 100 to 300 °C.

4. Process according to at least one of the preceding claims, **characterized in that** step A) is carried out at a pressure of from 5 to 100 bar.

5. Process according to at least one of the preceding claims, **characterized in that** the heterogeneous oxidation catalyst used according to step B) for the oxidative esterification reaction comprises one or more ultra-finely dispersed metals with an average particle size of < 20 nm selected from the group consisting of gold, palladium, ruthenium, rhodium and silver.

6. Process according to at least one of the preceding claims, **characterized in that** the heterogeneous oxidation catalyst used according to step B) for the oxidative esterification reaction comprises one or more members of the group consisting of lithium, sodium, potassium, calcium, magnesium, scandium, yttrium, lanthanum and other lanthanoids with atomic numbers from 58 to 71, silicon, titanium, zirconium, hafnium, vanadium, niobium, tantalum, chromium, molybdenum, tungsten, manganese, rhenium, iron, ruthenium, osmium, cobalt, rhodium, iridium, nickel, palladium, platinum, copper, silver, gold, zinc, cadmium, boron, aluminium, gallium, indium, thallium, germanium, tin, lead, antimony, bismuth, tellurium, these being respectively present in metallic and/or oxidized form.

7. Process according to at least one of Claims 2 to 6, **characterized in that** the reaction of propanal with formaldehyde to give methacrolein according to step A) uses a secondary amine as organic base.

8. Process according to at least one of Claims 2 to 7, **characterized in that** step A) uses at least one organic acid.

9. Process according to at least one of Claims 2 to 8, **characterized in that** the molar ratio of acid to organic base is in the range from 20:1 to 1:20.

10. Process according to at least one of the preceding claims, **characterized in that** the reaction according to step A) is carried out with a residence time in the range from 0.1 to 300 seconds.

11. Process according to at least one of the preceding claims, **characterized in that** the reaction according to step B) is carried out with a pressure in the range from 2 to 50 bar.

12. Process according to at least one of the preceding claims, **characterized in that** the oxidative esterification reaction according to step B) is carried out with a temperature in the range from 10 to 200 °C.

13. Process according to at least one of the preceding claims, **characterized in that** the oxidative esterification reaction according to step B) takes place with a molar ratio of methanol to methacrolein in the range from 1:1 to 50:1.

14. Process according to at least one of the preceding claims, **characterized in that** steps A) and B) are carried out in a continuous process.

15. Process according to at least one of the preceding claims, **characterized in that** the reactor volume in step A) is smaller than the reactor volume in step B) and the ratio of the two reactor volumes is in the range from 1:1000 to 1:100.

16. Process according to at least one of the preceding claims, **characterized in that** the total amount of separately added water in the two steps A) and B) during the conduct of the reaction is not greater than 100 mol%, based on methacrolein.

17. Process according to at least one of the preceding claims, **characterized in that** no water at all is separately added in the two steps A) and B) during the conduct of the reaction.

**Revendications**

1. Procédé de fabrication de méthacrylate de méthyle, comprenant les étapes suivantes :

A) la fabrication de méthacroléine à partir de propanal et de formaldéhyde, et
B) la mise en réaction de la méthacroléine obtenue à l'étape A) dans une réaction d'estérification oxydative pour former du méthacrylate de méthyle,

**caractérisé en ce que** les deux étapes A) et B) ont lieu dans la phase liquide à une pression de 2 à 100 bar, et l'étape B) est réalisée en présence d'un catalyseur hétérogène, comprenant des métaux et/ou des oxydes de métaux, contenant des métaux nobles.

2. Procédé selon la revendication 1, **caractérisé en ce que** l'étape A) est réalisée en présence de 0,1 à 20 % en moles d'une base organique et 0,1 à 20 % en moles d'un acide, à chaque fois par rapport au propanal.

3. Procédé selon la revendication 1 ou 2, **caractérisé en ce que** l'étape A) est réalisée à une température de 100 à 300 °C.

4. Procédé selon au moins l'une quelconque des revendications précédentes, **caractérisé en ce que** l'étape A) est réalisée à une pression de 5 à 100 bar.

5. Procédé selon au moins l'une quelconque des revendications précédentes, **caractérisé en ce que** le catalyseur d'oxydation hétérogène utilisé pour la réaction d'estérification oxydative selon l'étape B) comprend un ou plusieurs métaux dispersés ultrafinement ayant une taille de particule moyenne < 20 nm, qui sont choisis dans le groupe constitué par l'or, le palladium, le ruthénium, le rhodium et l'argent.

6. Procédé selon au moins l'une quelconque des revendications précédentes, **caractérisé en ce que** le catalyseur d'oxydation hétérogène utilisé pour la réaction d'estérification oxydative selon l'étape B) comprend un ou plusieurs membres du groupe constitué par le lithium, le sodium, le potassium, le calcium, le magnésium, le scandium, l'yttrium, le lanthane et les autres lanthanides de numéros atomiques 58 à 71, le silicium, le titane, le zirconium, l'hafnium, le vanadium, le niobium, le tantale, le chrome, le molybdène, le tungstène, le manganèse, le rhénium, le fer, le ruthénium, l'osmium, le cobalt, le rhodium, l'iridium, le nickel, le palladium, le platine, le cuivre, l'argent, l'or, le zinc, le cadmium, le bore, l'aluminium, le gallium, l'indium, le thallium, le germanium, l'étain, le plomb, l'antimoine, le bismuth, le tellure, qui se présentent chacun sous forme métallique et/ou oxydée.

7. Procédé selon au moins l'une quelconque des revendications 2 à 6, **caractérisé en ce que** la réaction du propanal avec le formaldéhyde pour former la méthacroléine selon l'étape A) a lieu en utilisant une amine secondaire en tant que base organique.

8. Procédé selon au moins l'une quelconque des revendications 2 à 7, **caractérisé en ce qu'**au moins un acide organique est utilisé à l'étape A).

9. Procédé selon au moins l'une quelconque des revendications 2 à 8, **caractérisé en ce que** le rapport molaire entre l'acide et la base organique se situe dans la plage allant de 20:1 à 1:20.

10. Procédé selon au moins l'une quelconque des revendications précédentes, **caractérisé en ce que** la réaction selon l'étape A) est réalisée avec un temps de séjour dans la plage allant de 0,1 à 300 secondes.

11. Procédé selon au moins l'une quelconque des revendications précédentes, **caractérisé en ce que** la réaction selon l'étape B) est réalisée à une pression dans la plage allant de 2 à 50 bar.

12. Procédé selon au moins l'une quelconque des revendications précédentes, **caractérisé en ce que** la réaction d'estérification oxydative selon l'étape B) a lieu à une température dans la plage allant de 10 à 200 °C.

13. Procédé selon au moins l'une quelconque des revendications précédentes, **caractérisé en ce que** la réaction d'estérification oxydative selon l'étape B) a lieu avec un rapport molaire entre le méthanol et la méthacroléine dans la plage allant de 1:1 à 50:1.

14. Procédé selon au moins l'une quelconque des revendications précédentes, **caractérisé en ce que** les étapes A) et B) sont réalisées par un procédé continu.

15. Procédé selon au moins l'une quelconque des revendications précédentes, **caractérisé en ce que** le volume du réacteur à l'étape A) est inférieur au volume du réacteur à l'étape B) et le rapport entre les deux volumes de réacteur se situe dans la plage allant de 1:1 000 à 1:100.

16. Procédé selon au moins l'une quelconque des revendications précédentes, **caractérisé en ce que** la quantité totale de l'eau ajoutée séparément dans les deux étapes A) et B) pendant la réalisation de la réaction n'est pas supérieure à 100 % en moles par rapport à la méthacroléine.

17. Procédé selon au moins l'une quelconque des revendications précédentes, **caractérisé en ce qu'**aucune eau n'est ajoutée séparément dans les deux étapes A) et B) pendant la réalisation de la réaction.

Fig. 1: Schematisches Verfahrensflussdiagramm

Fig. 2: Mögliches Verfahrensfließbild zu Schritt A)

EP 2 986 589 B1

Fig. 3: Mögliches Verfahrensfließbild zu Schritt B)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5969178 A
- US 7012039 B
- CN 101074192
- US 4408079 A
- EP 0890569 A
- EP 0890069 A
- EP 0092097 A
- DE 2855504
- US 7141702 B
- DE 3213681 A1
- US 2848499 A
- JP 4173757 A
- JP 19900300135 B
- JP 3069420 B
- EP 0317909 A2
- WO 2010030339 A1
- WO 2008071508 A1
- EP 982314 B1
- WO 2008012128 A1
- WO 2008006633 A1
- WO 2007036424 A1
- WO 2007028660 A1
- WO 2005090276 A1
- EP 1294668 B1
- US 7141702 B2
- EP 0857512 A1
- EP 1393800 A1
- EP 2177267 A1
- EP 2210664 A1
- US 4249019 A
- DE 3018071 A1
- US 7012039 B2
- EP 0376117 B1
- US 20020188151 A1
- CN 101074192 A1

### In der Beschreibung aufgeführte Nicht-Patentliteratur

- **YUCHAO LI et al.** Synthesis of methacrolein by condensation of propionaldehyde with formaldehyde. *Advance Materials Research,* 2012, vol. 396-398, 1094-1097

- **DESHPANDE et al.** Biphasic catalysis for a selective oxo-Mannich tandem synthesis of methacrolein. *Journal of Molecular Catalysis A: Chemical,* 2004, vol. 211, 49-53